# EUROPEAN PATENT APPLICATION

(11) **EP 3 498 273 A1**
(43) Date of publication of application: **19.06.2019**
(21) Application number: 17207291.0
(22) Date of filing: 14.12.2017
(51) Int. Cl.: A61K 31/277, A61P 25/00, A61P 25/24, A61P 25/20, A61P 25/22, A61P 25/06, A61P 25/08

(54) **PHARMACEUTICAL COMPOSITION FOR MODULATING THE RESPONSE OF A GABA-A RECEPTOR**

(71) Applicant: Universität Wien, 1010 Vienna (AT)
(72) Inventor: HERING, Steffen, 1190 Wien (AT); STADLER, Marco Johannes, 1060 Wien (AT); LANGER, Thierry, 1170 Wien (AT); SEIDEL, Thomas, 2721 Bad Fischau-Brunn (AT); MONTICELLI, Serena, 1150 Wien (AT); PACE, Vittorio, 1090 Wien (AT)
(74) Representative: Glas, Holger

(57) **Abstract**

The present invention relates to a pharmaceutical composition for use as a medicament and to the use of the pharmaceutical composition for modulating the response of a GABA_{A} receptor and the treatment of conditions like insomnia, anxiety, cardiac disease and/or epilepsy. Furthermore, the present invention refers to new active agents suitable for said pharmaceutical compositions.

## Description

### FIELD OF THE INVENTION

The present invention relates to a pharmaceutical composition for use as a medicament and to the use of the pharmaceutical composition for modulating the response of a GABA_{A} receptor and the treatment of conditions like insomnia, anxiety, cardiac disease and/or epilepsy. Furthermore, the present invention refers to new active agents suitable for said pharmaceutical compositions.

### BACKGROUND OF THE INVENTION

Epilepsy is one of the most common chronic neurological diseases worldwide. It is estimated that 1% of the world's population is affected by epilepsy during their lifetime with approximately 2.4 million new diagnosis being made each year. The disease is usually characterized by recurrent seizures, which may be defined as episodes of involuntary movement of a part or the entire body of a patient. Such epileptic seizures may vary in degree of severity from muscle jerks, shaking or trembling to severe and prolonged convulsions, which can lead to unconsciousness. Especially patients with strong convulsive seizures risk further injuries such as fractures and hematoma caused by the uncontrolled body movements or as a result of an accident during unconsciousness. In addition thereto, patients suffering from epilepsy often have psychological conditions such as anxiety or depression which may be based on e.g. social stigmata or stress caused by the unpredictability of the seizure events. Although, several antiepileptic and anticonvulsant drugs are available for subscription, a significant number of patients continue to have seizures despite such treatment or are resistant to the medicaments.

From a neurological point of view, it is believed that epilepsy is based on an excessive electrical discharge in nerve cells or larger neuronal networks, which distorts at least a part of the brain's neurological activity and ultimately leads to seizure symptoms. One of the reasons for these abnormal brain activities is a malfunction of the GABA_{A} receptors in nerve cells.

GABA_{A} receptors are ligand-gated ion channels that play a crucial role in regulating the electrochemical potential of nerve cells throughout the brain. The regulation is mediated by an interaction of the receptor with gamma-aminobutyric acid (GABA), the principal inhibitory neurotransmitter in the brain, which leads to a release of chloride ions into the intracellular matrix. This release alters the membrane potential of the nerve cell and can eventually inhibit the firing of cell signal in form of an action potential.

Modulating the response of the GABA_{A} receptors by interaction with small molecule drugs is one of the strategies for the treatment of epilepsy. For such an approach, the biochemical composition and the molecular structure of the targeted GABA_{A} receptors are particularly important. GABA_{A} receptors exist in the form of different subtypes each comprising five protein subunits. Up to now, 19 isoforms of mammalian GABA_{A} receptor subunits could be identified: α1-6, β1-3, γ1-3, δ, ε, π1-3, ρ and σ, and it seems that most of the endogenous as well as exogenous ligands bind to interfaces between two of the subunits. The GABA_{A} receptor subtypes often show a highly specific cellular and subcellular distribution in the brain. Therefore, it is theoretically possible to selectively target specific brain circuits by developing drugs that exhibit a high affinity for a specific GABA_{A} receptor subtype. This could increase the selectivity of a pharmaceutical for a certain brain area and may reduce severe side effects that can occur, for example, by the administration of known anticonvulsant drugs such as benzodiazepines.

Besides epilepsy, GABA_{A} receptors are implicated in cellular signaling processes involved in other diseases such as insomnia and anxiety, but also processes involved in pain, mood and panic disorders, schizophrenia and symptoms connected to alcohol and/or substance withdrawal/abuse. Therefore, compounds affecting GABA_{A} receptors, and specifically certain subtypes thereof, are suitable drug candidates for treating pain, mood and panic disorders, schizophrenia and symptoms connected to alcohol and/or substance withdrawal/abuse.

Furthermore, such compounds may be used for the diagnosis and treatment of anxiety, Down Syndrome, sleep and cognitive disorders, depression, overdose with benzodiazepine drugs, enhancement of memory and alertness, Huntington's Chorea, muscular spasms and rigidity, sleep and seizure disorders, and withdrawal symptoms. Antagonists may be used, for example, to diagnose and treat Alzheimer's disease, Parkinson's disease and for enhancing cognition and reversing sedation after application of general anesthesia during surgery. Modulators of GABA_{A} receptors can also be useful in the prevention of anxiety disorders, such as panic disorder with or without agoraphobia, agoraphobia without history of panic disorder, phobias including social phobias, obsessive-compulsive disorder, stress disorders including post-traumatic and acute stress disorder, and generalized or substance-induced anxiety disorder; depression or bipolar disorders such as single-episode or recurrent major depressive disorder, dysthymic disorder, bipolar I and bipolar II manic disorders; schizophrenia; attention deficit hyperactivity disorder and disorders of circadian rhythm, e.g. in subjects suffering from the effects of jet lag or shift work.

In view of the foregoing, there is a continuous need for alternative or improved pharmaceutical compositions containing compounds, which act as modulators of GABA_{A} receptors, and especially of specific subtypes thereof, and thus can be used for the treatment of specific conditions, like epilepsy.

### OBJECTIVE AND SUMMARY OF THE INVENTION

One objective of the present invention may be seen in the provision of an alternative or improved pharmaceutical composition based on compounds, which act as modulators of GABA_{A} receptors, and which are suitable for the treatment of diseases such as epilepsy, anxiety or insomnia.

Another objective of the present invention may be seen in the provision of new compounds, which exhibit an affinity for the GABA_{A} receptor. Another objective of the present invention may be seen in the provision of new compounds, which do not require a very complex synthesis as it is often the case for e.g. natural substances.

These and other objectives of the present invention, as they will become apparent from the ensuing description, are solved by the subject matter of the independent claims. The dependent claims relate to some preferred embodiments of the present invention.

According to one aspect of the present invention, a pharmaceutical composition for use as a medicament is provided, comprising a pharmaceutically effective amount of a compound of the general formula (I) 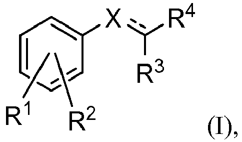 wherein
R¹ to R⁴ are independently selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups,
wherein R⁶ to R¹³ are independently selected from the group consisting of H, substituted or unsubstituted C₁ to C₆ alkyl and substituted or unsubstituted C₆ to C₁₄ aryl groups;
X is C-R⁵ or O, wherein R⁵ is selected from the group consisting of H, substituted or unsubstituted C₁ to C₆ alkyl groups and substituted or unsubstituted C₆ to C₁₄ aryl groups, wherein the dashed line represents an optional double bond, if X is C-R⁵;
   or a salt of said compound according to formula (I).

The inventors of the present invention surprisingly found that the specific compounds as defined in the present application and in the claims are suitable active agents for use in a medicament. More precisely, the new class of active agents according to formula (I) and formula (II) were found to provide a modulation of the GABA_{A} receptor response rendering the corresponding pharmaceutical compositions suitable for use as a medicament, e.g. for the treatment of conditions like insomnia, anxiety, cardiac disease and/or epilepsy.

According to one embodiment of the invention, the compound is selected from the general formula (II) 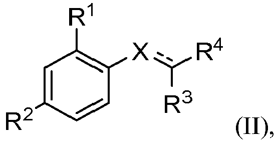 wherein
R¹ to R⁴ are independently selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups,
wherein R⁶ to R¹³ are independently selected from the group consisting of H, substituted or unsubstituted C₁ to C₆ alkyl and substituted or unsubstituted C₆ to C₁₄ aryl groups;
X is C-R⁵ or O, wherein R⁵ is selected from the group consisting of H, substituted or unsubstituted C₁ to C₆ alkyl groups and substituted or unsubstituted C₆ to C₁₄ aryl groups, wherein the dashed line represents an optional double bond, if X is C-R⁵;
or a salt of said compound according to formula (II).

According to another embodiment of the present invention, the compound is further specified in that
R¹ and R² are independently selected from the group consisting of H, F, Cl, Br, I, methyl, ethyl, propyl groups, butyl groups, pentyl groups and phenyl;
R³ and R⁴ are independently selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups;
and R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups, if X is C-R⁵.

According to one embodiment of the present invention, the compound according to formula (I) or (II) is further specified in that R¹ and R² are independently selected from the group consisting of F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl.

According to another preferred embodiment of the present invention, the compound according to formula (I) or (II) is further specified in that
R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R³ preferably is selected from the group consisting of H, methyl, -CN or -C(O)NR⁹R¹⁰ with R⁹ and R¹⁰ being both H, and most preferably R³ is -CN.

According to one embodiment of the present invention, the compound according to formula (I) or (II) is further specified in that
X is C-R⁵, wherein R⁵ is selected from the group consisting of H, methyl, ethyl, propyl groups, butyl groups, pentyl groups, trifluoromethyl and phenyl, preferably from the group consisting of H, methyl, ethyl, n-propyl, trifluoromethyl and phenyl, more preferably is H or methyl, and most preferably is methyl;
and in that the dashed line preferably represents a double bond.

According to one embodiment of the present invention, the compound according to formula (I) or (II) is further specified in that
in that R¹ and R² are independently selected from the group consisting of F, Cl and methyl and preferably are both Cl; and
in that R³ is -CN and R⁴ is selected from the group consisting of H, methyl, -CN, 1-tetrazole, -C(O)NR⁹R¹⁰, wherein R⁹ and R¹⁰ are both H, preferably R⁴ is selected from the group consisting H, -CN and -C(O)NR⁹R¹⁰, and most preferably is -CN,
or R⁴ is -CN and R³ is selected from the group consisting of H, methyl, -CN, 1-tetrazole, -C(O)NR⁹R¹⁰, wherein R⁹ and R¹⁰ are both H, preferably R³ is selected from the group consisting H, -CN and -C(O)NR⁹R¹⁰, and most preferably is -CN; and
in that X is C-R⁵, wherein R⁵ is selected from the group consisting of H, methyl, ethyl, n-propyl, trifluoromethyl and phenyl, more preferably from the group consisting of H and methyl, and most preferably is methyl; and
in that the dashed line preferably represents a double bond.

According to yet another embodiment of the present invention, the compound is selected from the group consisting of the following compounds 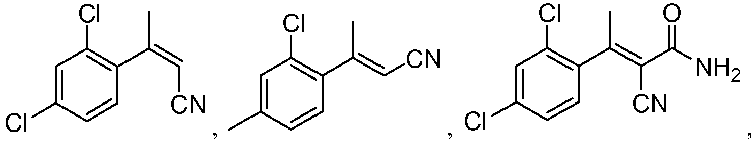 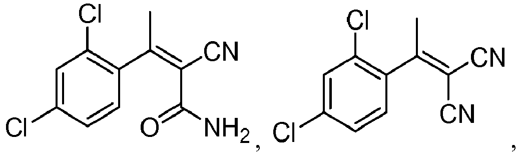 and salts thereof.

According to one embodiment of the present invention, the medicament or pharmaceutical composition is a GABA_{A} receptor response modulator, especially a positive allosteric GABA_{A} receptor modulator.

According to one embodiment of the present invention, the modulation of the GABA_{A} receptor response involves the interaction of the compound of the composition with the β subunit(s), especially the β2 and/or β3 isoform(s) of the β subunit(s) of the GABA_{A} receptor.

According to one embodiment of the present invention, the modulation of the GABA_{A} receptor response involves the interaction of the compound of the composition with the amino acid β_{2N265} of the β2 subunit and/or the amino acid β_{3N265} of the β3 subunit.

According to another aspect of the present invention, a compound of the general formula (I) 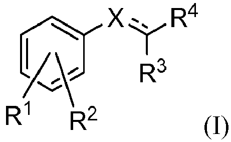 is provided, wherein
R¹ is Cl and R² is selected from the group consisting of halogen atoms, substituted or unsubstituted C₁ to C₆ alkyl groups and substituted or unsubstituted C₆ to C₁₄ aryl groups; and
wherein R³ is -CN and R⁴ is selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups, wherein R⁶ to R¹³ are independently selected from the group consisting of H, substituted or unsubstituted C₁ to C₆ alkyl and substituted or unsubstituted C₆ to C₁₄ aryl groups,
or R⁴ is -CN and R³ is selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups, wherein R⁶ to R¹³ are independently selected from the group consisting of H, substituted or unsubstituted C₁ to C₆ alkyl and substituted or unsubstituted C₆ to C₁₄ aryl groups; and
wherein X is C-R⁵, wherein R⁵ is selected from the group consisting of H, substituted or unsubstituted C₁ to C₆ alkyl groups and substituted or unsubstituted C₆ to C₁₄ aryl groups; and
wherein the dashed line represents a double bond;
or a salt of said compound according to formula (I).

The specific compounds as defined according to the foregoing aspect of the present invention are not only suitable active agents for use in a medicament, but also represent a new class of chemical compounds. It is noted that the defined compounds according to the foregoing aspect, i.e. the new class of chemical compounds represents a subgroup of the compounds as defined in claim 1 of the present application relating to a medicament or pharmaceutical composition. Also this new class of compounds according to formula (I) and formula (II) was found to provide a modulation of the GABA_{A} receptor response rendering the corresponding pharmaceutical compositions suitable for use as a medicament, e.g. for the treatment of conditions like insomnia, anxiety, cardiac disease and/or epilepsy.

According to one embodiment of the new class of compounds according to the present invention, the compound is selected from the general formula (II) 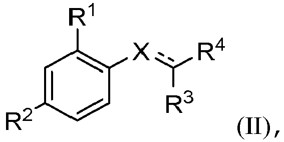 wherein
R¹ is Cl and R² is selected from the group consisting of halogen atoms, substituted or unsubstituted C₁ to C₆ alkyl groups and substituted or unsubstituted C₆ to C₁₄ aryl groups; and
wherein R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups,
or R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups; and
wherein X is C-R⁵, wherein R⁵ is selected from the group consisting of H, substituted or unsubstituted C₁ to C₆ alkyl groups and substituted or unsubstituted C₆ to C₁₄ aryl groups; and
wherein the dashed line represents a double bond;
or a salt of said compound according to formula (II).

According to yet another embodiment of the present invention, the new class of compounds is further specified in that
R² is selected from the group consisting of halogen atoms or unsubstituted C₁ to C₆ alkyl groups, preferably is Cl or methyl, and most preferably is Cl; and
wherein R³ is -CN and R⁴ is selected from the group consisting of H, methyl, -CN, 1-tetrazole, -C(O)NR⁹R¹⁰, wherein R⁹ and R¹⁰ are both H, preferably R⁴ is selected from the group consisting of H, -CN and -C(O)NR⁹R¹⁰, and most preferably R⁴ is -CN,
or R⁴ is -CN and R³ is selected from the group consisting of H, methyl, -CN, 1-tetrazole, -C(O)NR⁹R¹⁰, wherein R⁹ and R¹⁰ are both H, preferably R³ is selected from the group consisting of H, -CN and -C(O)NR⁹R¹⁰, and most preferably R³ is -CN; and
wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups.

According to yet another aspect of the invention, a pharmaceutical composition as described herein is provided for treating pain, mood and panic disorders, insomnia, anxiety, and/or neurologic disease, especially migraine and/or epilepsy.

### DETAILED DESCRIPTION OF THE PRESENT INVENTION

In the following the invention as defined hereinabove will be described in more detail. It is to be understood that any definitions, embodiments and descriptions referred to herein apply to all aspects of the present invention, i.e. to the pharmaceutical composition for use as a medicament, the compounds as well for the pharmaceutical composition for treating the specified conditions.

### Definitions

It should be understood that for the purposes of the present invention, the following terms have the following meanings:
A "pharmaceutical composition" in the meaning of the present invention refers to the combination of one or more compounds (e.g., compounds of formula (I) or (II) and compounds specifically described herein) that may be used for treating a subject in need of treatment, optionally comprising one or more pharmaceutically acceptable excipients.

A "pharmaceutically acceptable excipient" in the meaning of the present invention can be any substance used for the preparation of pharmaceutical dosage forms, including but not limited to coating materials, film-forming materials, fillers, disintegrating agents, release-modifying materials, carrier materials, diluents, binding agents and other adjuvants.

A "pharmaceutically effective amount" of a compound as described in the present invention refers to the quantity of the compound that may be used for treating a subject and may depend on the weight and age of the subject and the route of administration, among other things.

The term "halogen atom" includes fluoride, bromide, chloride or iodide. The term "halo" means -F, -Cl, -Br or -I. A "haloalkyl" group in the meaning of the present invention is an alkyl group as described herein, wherein at least one hydrogen atom is substituted by a halogen atom. Exemplary haloalkyl groups include a pentafluoroethyl or trifluoromethyl group.

The term "alkyl" refers to straight chain and branched saturated hydrocarbon groups, having a specified number of carbon atoms (e.g., C₁ to C₆ alkyl refers to an alkyl group having 1 to 6 carbon atoms). Examples of alkyl groups include methyl, ethyl, *n*-propyl, *i*-propyl, *n*-butyl, *s*-butyl, *i*-butyl, *t*-butyl, pent-1-yl, pent-2-yl, pent-3-yl, 3-methylbut-1-yl, 3-methylbut-2-yl, 2-methylbut-2-yl, 2,2,2-trimethyleth-1-yl, *n*-hexyl, and the like. When the expression "propyl groups" is used herein, it is meant to encompass *n*-propyl, *i*-propyl, when the expression "butyl groups" is used herein, it is meant to encompass *n*-butyl, *s*-butyl, *i*-butyl, *t*-butyl, and so on.

The term "cycloalkyl" refers to saturated monocyclic hydrocarbon groups, generally having a specified number of carbon atoms that comprise the ring or rings (e.g., C₃ to C₆ cycloalkyl refers to a cycloalkyl group having 3 to 6 carbon atoms as ring members). The cycloalkyl group may be attached to a parent group or to a substrate at any ring atom unless such attachment would violate valence requirements. Examples of monocyclic cycloalkyl groups include cyclopropyl, cyclobutyl, cyclopentyl and cyclohexyl.

The term "aryl" in the meaning of the present invention refers to fully unsaturated monocyclic aromatic hydrocarbons and to polycyclic hydrocarbons having at least one aromatic ring, both monocyclic and polycyclic aryl groups generally having a specified number of carbon atoms that comprise their ring members (e.g., C₆ to C₁₄ aryl refers to an aryl group having 6 to 14 carbon atoms as ring members). The aryl group may be attached to a parent group or to a substrate at any ring atom and may include one or more non-hydrogen substituents unless such attachment or substitution would violate valence requirements. Examples of aryl groups include phenyl, biphenyl, cyclobutabenzenyl, indenyl, naphthalenyl, benzocycloheptanyl, biphenylenyl, fluorenyl, and the like.

The term "substituted" when used in connection with a chemical substituent or moiety (e.g., an alkyl group), means that one or more hydrogen atoms of the substituent or moiety have been replaced with one or more non-hydrogen atoms or groups. The term particularly refers to substituted moieties (including substituted C₁ to C₆ alkyl groups, substituted C₃ to C₆ cycloalkyl groups, substituted C₆ to C₁₄ aryl groups, or substituted heterocyclic groups) bearing one or more of the following groups or substituents: halogen, -C₁-C₆ alkyl, -C₁-C₆ alkenyl, -hydroxyl, -NH₂,-NH(C₁-C₆ alkyl), -N(C₁-C₆ alkyl)(C₁-C₆ alkyl), -N(C₁-C₃ alkyl)C(O)(C₁-C₆ alkyl),-NHC(O)(C₁-C₆ alkyl), -NHC(O)H, -C(O)NH₂, -C(O)NH(C₁-C₆ alkyl), -C(O)N(C₁-C₆ alkyl)(C₁-C₆ alkyl), -CN, CHN(C₁-C₆ alkyl), -O(C₁-C₆ alkyl), -C(O)OH, -C(O)O(C₁-C₆ alkyl), -(C₁-C₆ alkyl)C(O)O(C₁-C₆ alkyl), -C(O)(C₁-C₆ alkyl), -C₆-C₁₄ aryl, -C₅-C₉ heteroaryl, -C₃-C₈ cycloalkyl, -haloalkyl, -aminoalkyl, -OC(O)(C₁-C₆ alkyl), -C₁-C₆ carboxyamidoalkyl and/or -NO₂.

A "heterocyclic" group in the meaning of the present invention refers to saturated or partially unsaturated monocyclic groups having ring atoms composed of carbon atoms and 1 to 4 heteroatoms independently selected from nitrogen, oxygen, and sulfur. The monocyclic group generally has a specified number of carbon atoms in their ring or rings (e.g., C₂ to C₄ heterocyclic group refers to a heterocyclic group having 2 to 5 carbon atoms and 1 to 4 heteroatoms as ring members). The heterocyclic group may be attached to a parent group or to a substrate at any ring atom and may include one or more non-hydrogen substituents unless such attachment or substitution would violate valence requirements or result in a chemically unstable compound. Examples of heterocyclic groups include tetrazolyl, imidazolyl, pyrazol, aziridinyl (e.g., aziridin-1-yl and aziridin-2-yl), tetrahydrofuranyl, pyrrolidinyl, tetrahydropyranyl, tetrahydrothiopyranyl, piperidinyl, 1,4-dioxanyl, 1,2,3,4-tetrahydropyridinyl, and 1,2,5,6-tetrahydropyridinyl.

Some of the compounds disclosed herein may contain one or more asymmetric centers and may thus lead to enantiomers, diastereomers, and other stereoisomeric forms.

The present invention is also meant to encompass all such possible forms as well as their racemic and resolved forms and mixtures thereof, unless specified otherwise. When the compounds described herein contain olefinic double bonds or other centers of geometric asymmetry, and unless specified otherwise, it is intended to include both *E* and *Z* geometric isomers. All tautomers are intended to be encompassed by the present invention as well.

As used herein, the term "stereoisomers" is a general term for all isomers of individual molecules that differ only in the orientation of their atoms in space. It includes enantiomers and isomers of compounds with more than one chiral center that are not mirror images of one another, or non-superimposable olefins without a chiral center (diastereomers).

The term "enantiomer" or "enantiomeric" refers to a molecule that is nonsuperimposeable on its mirror image and hence optically active wherein the enantiomer rotates the plane of polarized light in one direction and its mirror image rotates the plane of polarized light in the opposite direction.

The expression "salt of said compound" or "salt thereof" is meant to encompass all pharmaceutically acceptable salts of the disclosed compounds. The pharmaceutically acceptable salts include, but are not limited to, metal salts such as sodium salt, potassium salt, cesium salt and the like; alkaline earth metals such as calcium salt, magnesium salt and the like; organic amine salts such as triethylamine salt, pyridine salt, picoline salt, ethanolamine salt, triethanolamine salt, dicyclohexylamine salt, N,N'-dibenzylethylenediamine salt and the like; inorganic acid salts such as hydrochloride, hydrobromide, sulfate, phosphate and the like; organic acid salts such as formate, acetate, trifluoroacetate, maleate, fumarate, tartrate and the like; sulfonates such as methanesulfonate, benzenesulfonate, p-toluenesulfonate, and the like; amino acid salts such as arginate, asparginate, glutamate and the like.

The compounds of the present invention are useful for modulating a pharmacodynamic response from GABA_{A} receptors. The response can be attributed to the compound stimulating (agonist) or inhibiting (antagonist) the GABA_{A} receptors. This can depend on the subunit composition of the GABA_{A} receptors.

The term "modulate" as used herein with respect to the GABA_{A} receptors means the mediation of a pharmacodynamic response (e.g. a sedative effect) in a subject from (i) inhibiting or activating the receptor, or (ii) directly or indirectly affecting the normal regulation of the receptor activity, i.e. by binding to an allosteric binding site of the receptor. Compounds which mediate a pharmacodynamic response as explained above include agonists, antagonists, mixed agonists/antagonists and compounds which directly or indirectly affect regulation of the receptor activity, such as an allosteric modulator.

Where the term "comprising" is used in the present description and claims, it does not exclude other elements. For the purposes of the present invention, the term "consisting of" is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e.g. "a", "an" or "the", this includes a plural of that noun unless something else is specifically stated.

### The pharmaceutical composition

According to one aspect of the present invention, a pharmaceutical composition for use as a medicament is provided, comprising a pharmaceutically effective amount of a compound of the general formula (I) 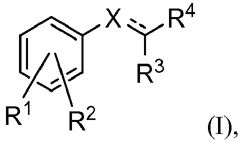 wherein
R¹ to R⁴ are independently selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups,
wherein R⁶ to R¹³ are independently selected from the group consisting of H, substituted or unsubstituted C₁ to C₆ alkyl and substituted or unsubstituted C₆ to C₁₄ aryl groups;
X is C-R⁵ or O, wherein R⁵ is selected from the group consisting of H, substituted or unsubstituted C₁ to C₆ alkyl groups and substituted or unsubstituted C₆ to C₁₄ aryl groups, wherein the dashed line represents an optional double bond, if X is C-R⁵;
or a salt of said compound according to formula (I).

The inventors surprisingly found out that compounds of the general formula (I) exhibit an affinity for the GABA_{A} receptor. The compounds can thus be used in a pharmaceutical composition for treating diseases which are related to the GABA_{A} receptor signaling pathway or diseases which can be treated by modulating the GABA_{A} receptor response. Examples of such diseases are epilepsy, anxiety or insomnia. The corresponding class of compounds according to formula (I) not only provides excellent activities but are also easily accessible via standard chemical procedures or synthesis. The inventors inter alia found that the inventive pharmaceutical compositions modulate the receptor response by involving the interaction of the compound of the composition with the amino acid β_{3N265} of the β3 subunit, which allows for the treatment of the conditions set out herein.

In the following, the pharmaceutical compositions containing compounds being suitable according the present invention will be defined in more detail. Especially suitable or preferred subgroups of said compounds will be defined.

It is supposed that the disubstituted phenyl group of the compound according to formula (I) or (II) represents an ensemble of steric and electronic features that is advantageous for ensuring the optimal supramolecular interactions with GABA_{A} receptor and to modulate its biological response. Without wishing to be bound by theory, it is hypothesized that the benzene core of the disubstituted phenyl group interacts inter alia with the benzene core of phenylalanine F289 of subunit β2 and/or β3, especially β3, via π-stacking, i.e. an interaction of the π- electron system of the aromatic rings. In this context, it has further been shown that compounds according to formula (I) or (II) bearing R¹ and R² substituents independently selected from the group consisting of methyl and chloride are especially effective for interacting with the GABA_{A} receptor, and potentially with a hydrophobic pocket thereof. Thus, substitution pattern falling within this group may be considered to be privileged motifs. Compounds according to formula (II) with a chloro-substituent as R¹ and R² seem to be particularly suited for the interaction with subunits of the GABA_{A} receptor.

The compound according to formula (I) according to one embodiment of the present invention is further specified in that
R¹ and R² are independently selected from the group consisting of Cl and methyl, and most preferably are both Cl,
in that R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R³ preferably is selected from the group consisting of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents an optional double bond, if X is C-R⁵;
or a salt of said compound according to formula (I).

It turned out that a CN group as substituent R³ or R⁴ positively influences the affinity and/or interaction for/with the GABA_{A} receptor. In case, a CN group is present for substituents R³ and R⁴ the modulation of the GABA_{A} receptor response may be further promoted. These observations show that a nitrile group for R³ and/or R⁴ may be supportive for the biological activity of the compound. A reason for the increased affinity of such compounds may be a hydrogen-bond donor/acceptor interaction between a functional residue and/or the peptide bond of an amino acid and the nitrogen of the nitrile group(s). Amino acids, which are particularly known to act as a hydrogen-bond donor, are, for example, histidine, tyrosine, asparagine or serine. Mutation studies have suggested that the nitrile group(s) of the compound might form a hydrogen bond to the carbamoyl group of the amino acid β_{2N265} of the β2 subunit and/or the amino acid β_{3N265} of the β3 subunit of the GABA_{A} receptor, and especially to the amino acid β_{3N265} of the β3 subunit of the GABA_{A} receptor.

According to another embodiment of the present invention, the compound according to formula (I) is further specified in that
R¹ and R² are independently selected from the group consisting of H, F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl;
in that R³ is -CN and R⁴ is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, methyl, -CN or -C(O)NR⁹R¹⁰ with R⁹ and R¹⁰ being both H, and most preferably R³ is CN;
X is C-R⁵ or O, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents an optional double bond, if X is C-R⁵;
or a salt of said compound according to formula (I).

It has also been found that an increased planarity and/or a decreased steric flexibility of the compound according to formula (I) or (II) may be advantageous for the modulation of the GABA_{A} receptor response. For example, if X is C-R⁵ and the C atom is sp² hybridized, i.e. forms part of a double bond, the modulation of the GABA_{A} receptor may be more pronounced. Furthermore, it appears that the hydrophobicity of substituent R⁵ may be another parameter for influencing the biological activity of the compound according to formula (I) or (II). In this context, it seems that a more hydrophobic substituent R⁵ may lead to a stronger interaction of the compound and the GABA_{A} receptor. This could be an indication that this residue of the molecule interacts with a hydrophobic "pocket" of the GABA_{A} protein complex.

According to another embodiment of the present invention, the compound according to formula (I) is further specified in that
R¹ and R² are independently selected from the group consisting of Cl and methyl, and most preferably are both Cl;
in that R³ is -CN and R⁴ is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, methyl, -CN or -C(O)NR⁹R¹⁰ with R⁹ and R¹⁰ being both H, and most preferably R³ is CN;
X is C-R⁵, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents a double bond;
or a salt of said compound according to formula (I).

The compound according to formula (I) is preferably further specified in that
R¹ and R² are independently selected from the group consisting of H, F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl;
in that R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R³ preferably is selected from the group consisting of H, methyl, -CN or -C(O)NR⁹R¹⁰ with R⁹ and R¹⁰ being both H, and most preferably R³ is -CN;
X is C-R⁵, wherein R⁵ is H or methyl, and most preferably is methyl,
wherein the dashed line represents a double bond;
or a salt of said compound according to formula (I).

According to another preferred embodiment of the present invention, the compound according to formula (I) is further specified in that
R¹ and R² are independently selected from the group consisting of Cl and methyl, and most preferably are both Cl,
in that R³ is -CN or -C(O)NR⁹R¹⁰ with R⁹ and R¹⁰ being both H and R⁴ is selected from the group of H, methyl, -CN or -C(O)NR⁹R¹⁰ with R⁹ and R¹⁰ being both H, and most preferably R⁴ is -CN;
or in that R⁴ is -CN or -C(O)NR⁹R¹⁰ with R⁹ and R¹⁰ being both H and R³ is selected from the group of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, methyl, ethyl, n-propyl, trifluoromethyl and phenyl, more preferably is H or methyl, and most preferably is methyl;
wherein the dashed line represents an optional double bond, if X is C-R⁵;
or a salt of said compound according to formula (I).

According to yet another embodiment of the present invention, the compound according to formula (I) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
or in that R⁴ is -CN and R³ is selected from the group of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, methyl, ethyl, n-propyl, trifluoromethyl and phenyl, more preferably is H or methyl, and most preferably is methyl;
wherein the dashed line represents an optional double bond, if X is C-R⁵;
or a salt of said compound according to formula (I).

According to certain embodiments of the present invention, the compound according to formula (I) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
or in that R⁴ is -CN and R³ is selected from the group of H, methyl, -CN or -C(O)NR⁹R¹⁰ with R⁹ and R¹⁰ being both H, and most preferably R³ is -CN;
X is C-R⁵, wherein R⁵ is selected from the group consisting of H, methyl, ethyl, n-propyl, trifluoromethyl and phenyl, more preferably is H or methyl, and most preferably is methyl;
wherein the dashed line represents a double bond;
or a salt of said compound according to formula (I).

In case X is C-R⁵ and forms part of a double bond, the stereochemistry of the double bond can have a significant influence on the modulation have the GABA_{A} receptor response. For example, it has been shown that a CN group as substituent R³, i.e. in *cis*-position to the phenyl group, may be more preferred than as substituent R⁴, i.e. in *trans*-position to the phenyl group.

Thus, according to one embodiment, the compound according to formula (I) is specified in that
R¹ and R² are independently selected from the group consisting of H, F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl;
in that R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
X is C-R⁵, wherein R⁵ is selected from the group consisting of H, methyl, ethyl, n-propyl, trifluoromethyl and phenyl, more preferably is H or methyl, and most preferably is methyl,
wherein the dashed line represents a double bond;
or a salt of said compound according to formula (I).

According to certain embodiments of the present invention, the compound according to formula (I) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
X is C-R⁵, wherein R⁵ is selected from the group consisting of H, methyl, ethyl, n-propyl, trifluoromethyl and phenyl, more preferably is H or methyl, and most preferably is methyl;
wherein the dashed line represents a double bond;
or a salt of said compound according to formula (I).

According to further preferred embodiments of the present invention, the compound is selected from the general formula (II) 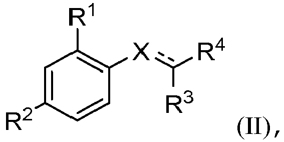 wherein
R¹ to R⁴ are independently selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups,
wherein R⁶ to R¹³ are independently selected from the group consisting of H, substituted or unsubstituted C₁ to C₆ alkyl and substituted or unsubstituted C₆ to C₁₄ aryl groups;
X is C-R⁵ or O, wherein R⁵ is selected from the group consisting of H, substituted or unsubstituted C₁ to C₆ alkyl groups and substituted or unsubstituted C₆ to C₁₄ aryl groups, wherein the dashed line represents an optional double bond, if X is C-R⁵;
or a salt of said compound according to formula (II).

According to one embodiment of the present invention, the compound according to formula (II) is further specified in that
R¹ and R² are independently selected from the group consisting of Cl and methyl, and most preferably are both Cl,
in that R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R³ preferably is selected from the group consisting of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents an optional double bond, if X is C-R⁵;
or a salt of said compound according to formula (II).

According to another inventive embodiment, the compound according to formula (II) is further specified in that
R¹ and R² are independently selected from the group consisting of H, F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl;
in that R³ is -CN and R⁴ is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents an optional double bond, if X is C-R⁵;
or a salt of said compound according to formula (II).

According to yet another embodiment of the present invention, the compound according to formula (II) is further specified in that
R¹ and R² are independently selected from the group consisting of H, F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl;
in that R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R³ preferably is selected from the group consisting of H, methyl, -CN or -C(O)NR⁹R¹⁰ with R⁹ and R¹⁰ being both H, and most preferably R³ is -CN;
X is C-R⁵, wherein R⁵ is H or methyl, and most preferably is methyl,
wherein the dashed line represents a double bond;
or a salt of said compound according to formula (II).

According to another embodiment of the present invention, the compound according to formula (II) is further specified in that
R¹ and R² are independently selected from the group consisting of Cl and methyl, and most preferably are both Cl;
in that R³ is -CN and R⁴ is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, methyl, -CN or -C(O)NR⁹R¹⁰ with R⁹ and R¹⁰ being both H, and most preferably R³ is CN;
X is C-R⁵, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents a double bond;
or a salt of said compound according to formula (II).

According to another embodiment, the compound according to formula (II) is specified in that
R¹ and R² are independently selected from the group consisting of H, F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl;
in that R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
X is C-R⁵, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups;
wherein the dashed line represents a double bond;
or a salt of said compound according to formula (I).

According an inventive embodiment, the compound according to formula (II) is further specified in that
R¹ and R² are independently selected from the group consisting of Cl and methyl, and most preferably are both Cl,
in that R³ is -CN or -C(O)NR⁹R¹⁰ with R⁹ and R¹⁰ being both H and R⁴ is selected from the group of H, methyl, -CN or -C(O)NR⁹R¹⁰ with R⁹ and R¹⁰ being both H, and most preferably R⁴ is -CN;
or in that R⁴ is -CN or -C(O)NR⁹R¹⁰ with R⁹ and R¹⁰ being both H and R³ is selected from the group of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, methyl, ethyl, n-propyl, trifluoromethyl and phenyl, more preferably is H or methyl, and most preferably is methyl;
wherein the dashed line represents an optional double bond, if X is C-R⁵;
or a salt of said compound according to formula (II).

According to one embodiment of the present invention, the compound according to formula (II) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
or in that R⁴ is -CN and R³ is selected from the group of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, methyl, ethyl, n-propyl, trifluoromethyl and phenyl, more preferably is H or methyl, and most preferably is methyl;
wherein the dashed line represents an optional double bond, if X is C-R⁵; or a salt of said compound according to formula (II).

According to one embodiment of the present invention, the compound according to formula (II) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
or in that R⁴ is -CN and R³ is selected from the group of H, methyl, -CN or -C(O)NR⁹R¹⁰ with R⁹ and R¹⁰ being both H, and most preferably R³ is -CN;
X is C-R⁵, wherein R⁵ is selected from the group consisting of H, methyl, ethyl, n-propyl, trifluoromethyl and phenyl, more preferably is H or methyl, and most preferably is methyl;
wherein the dashed line represents a double bond;
or a salt of said compound according to formula (II).

According to certain embodiments of the present invention, the compound according to formula (I) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
X is C-R⁵, wherein R⁵ is selected from the group consisting of H, methyl, ethyl, n-propyl, trifluoromethyl and phenyl, more preferably is H or methyl, and most preferably is methyl;
wherein the dashed line represents a double bond;
or a salt of said compound according to formula (I).

The pharmaceutical composition as described herein can be used as a medicament for modulating the GABA_{A} receptor response or for treating conditions in patients by modulating the GABA_{A} receptor response. Thus, according to one embodiment of the present invention, the medicament is a GABA_{A} receptor response modulator. According to a preferred embodiment, the medicament is a GABA_{A} receptor response activator. A "GABA_{A} receptor response activator" in the meaning of the present invention refers to a compound or a pharmaceutical composition based thereon which directly activates the GABA_{A} receptor by binding to the receptor as an agonist, or, alternatively, indirectly activates the GABA_{A} receptor as a positive allosteric modulator. A "positive allosteric modulator" is a compound inducing potentiation of the orthosteric agonist's effect, either by enhancing the binding affinity or the functional efficacy of the orthosteric agonist, for example GABA, for the target protein, for the target protein, for example the GABA_{A} receptor. According to a more preferred embodiment, the medicament is a positive allosteric modulator of the GABA_{A} receptor response, i.e. a positive allosteric GABA_{A} receptor modulator.

A medicament comprising a compound according to formula (I) or (II) leads to a potentiation of the GABA-induced chloride current (I_{GABA}) into the nerve cell, which leads to a hyperpolarization of the cell and thereby may prevent the firing of an action potential by the neuron. The resulting decreased activity of the neuronal network ultimately allows for treating specific conditions such as anxiety, panic disorder, insomnia and/or epilepsy. A particular high potentiation of the I_{GABA} by a compound according to formula (I) or (II) thus reflects its potential as a drug for treating conditions such as anxiety, panic disorder, insomnia and/or epilepsy. The above-described preferred embodiments for each substituent R¹ to R⁵ have the effect to potentiate the I_{GABA}. Thus, the selection of a preferred embodiment of one of the substituents R¹ to R⁵ or the selection of a combination of preferred embodiments of these substituents allows for designing potent drugs for treating the conditions described herein, and especially anxiety, panic disorder, insomnia and/or epilepsy.

It was shown that a compound according to formula (I) or (II), and especially the preferred embodiments thereof, have the effect on a subject, that the seizure threshold, i.e. the amount of stimuli needed until a subject suffers from seizure symptoms, is increased. Thus, the medicament according to the present invention is particularly suited for treating epilepsy.

According to another embodiment, the compound according to formula (I) or (II) is further specified in that
R¹ and R² are independently selected from the group consisting of F, Cl and methyl, even more preferably from the group consisting of Cl and methyl, and most preferably are both Cl;
in that R³ is -CN and R⁴ is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents an optional double bond, if X is C-R⁵;
or a salt of said compound according to formula (I) or (II),
wherein the medicament is used for treating anxiety, panic disorder, insomnia and/or epilepsy, and especially epilepsy.

According to another embodiment, the compound according to formula (I) or (II) is further specified in that
R¹ and R² are independently selected from the group consisting of F, Cl and methyl, even more preferably from the group consisting of Cl and methyl, and most preferably are both Cl;
in that R³ is -CN and R⁴ is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, methyl, -CN or -C(O)NR⁹R¹⁰ with R⁹ and R¹⁰ being both H, and most preferably R³ is -CN;
X is C-R⁵, wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups,
wherein the dashed line represents a double bond;
or a salt of said compound according to formula (I) or (II),
wherein the medicament is used for treating anxiety, panic disorder, insomnia and/or epilepsy, and especially epilepsy.

According to one embodiment, the compound according to formula (I) or (II) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
or in that R⁴ is -CN and R³ is selected from the group of H, methyl, -CN or -C(O)NR⁹R¹⁰ with R⁹ and R¹⁰ being both H, and most preferably R³ is -CN;
X is C-R⁵ or O, wherein R⁵ is selected from the group consisting of H, methyl, ethyl, n-propyl, trifluoromethyl and phenyl, more preferably is H or methyl, and most preferably is methyl;
wherein the dashed line represents an optional double bond, if X is C-R⁵;
or a salt of said compound according to formula (I) or (II),
wherein the medicament is used for treating anxiety, panic disorder, insomnia and/or epilepsy, and especially epilepsy.

According to one embodiment of the present invention, the compound according to formula (I) or (II) is further specified in that
R¹ and R² are independently selected from the group consisting of Cl and methyl, and most preferably are both Cl,
in that R³ is -CN and R⁴ is selected from the group of H, methyl, -CN or -C(O)NR⁹R¹⁰ with R⁹ and R¹⁰ being both H, and most preferably R⁴ is -CN;
or in that R⁴ is -CN and R³ is selected from the group of H, methyl, -CN or -C(O)NR⁹R¹⁰ with R⁹ and R¹⁰ being both H, and most preferably R³ is -CN;
X is C-R⁵, wherein R⁵ is selected from the group consisting of H, methyl, ethyl, n-propyl, trifluoromethyl and phenyl, more preferably is H or methyl, and most preferably is methyl;
wherein the dashed line represents a double bond;
or a salt of said compound according to formula (I) or (II),
wherein the medicament is used for treating anxiety, panic disorder, insomnia and/or epilepsy, and especially epilepsy.

The modulation of the GABA_{A} receptor response may involve the interaction of one or more specific subunit(s) of the GABA_{A} receptor with the compound of the pharmaceutical composition. According to one embodiment of the present invention, the modulation of the GABA_{A} receptor response involves the interaction of the compound of the composition with the β subunit(s) of the GABA_{A} receptor, and especially the β2 and/or β3 isoform(s) of the β subunit.

The compounds of the inventive pharmaceutical composition may modulate the GABA_{A} receptor response depending on the specific subtype of the GABA_{A} receptor. Thus, according to one embodiment, the modulation of the GABA_{A} receptor response involves the interaction of the compound of the composition with a specific subtype of the GABA_{A} receptor, and especially with a subtype of the GABA_{A} receptor comprising a β2 and/or β3 isoform(s) of the β subunit.

The inventors surprisingly found that the specific compounds according to the general formula (I) or (II) have a particularly high affinity for specific subtypes of GABA_{A} receptors. For example, the compound according to the general formula (I) or (II) shows a β2 and β3-subunit-dependent enhancement of GABA-induced chloride currents (IGABA), and thus can be used to selectively target GABA_{A} receptor comprising such subunits. Based thereon, it is possible to selectively modulate the GABA_{A} receptor response in specific areas of the brain or spinal cord where these subtypes of GABA_{A} receptors are predominant. Furthermore, selective targeting of certain subunits, for example β2/3 over β1, by a drug is generally acknowledged to result in a reduction of side effects affecting the patient.

According to one embodiment of the present invention, the modulation of the GABA_{A} receptor response involves the interaction of the compound of the composition with the amino acid β_{3N265} of the β₃ subunit.

According to one embodiment, the modulated response of GABA_{A} receptor(s) is/are the response of GABA_{A} receptor subtype(s) al β2γ2S and/or α1β3γ2S.

The effect provided by the inventive compounds, i.e. the modulation of the GABA receptor response allows for the treatment of specific conditions or disease in patients suffering from said conditions. Thus, according to another aspect of the present invention, a pharmaceutical composition comprising the inventive compounds is provided for treating pain, mood and panic disorders, insomnia, anxiety, cardiac disease, especially cardiac arrhythmia, and/or neurologic disease, especially migraine and/or epilepsy. The pharmaceutical composition as described herein may thus *inter alia* be used as analgesic, anesthetic, sedative, hypnotic, anxiolytic, antiepileptic.

According to a preferred embodiment, the pharmaceutical composition is used for treating insomnia, anxiety or epilepsy, and especially for treating epilepsy.

### The new class of compounds

According to another aspect of the present invention, a compound of the general formula (I) 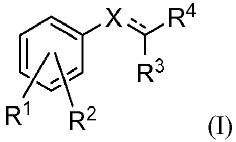 is provided, wherein
R¹ is Cl and R² is selected from the group consisting of halogen atoms, substituted or unsubstituted C₁ to C₆ alkyl groups and substituted or unsubstituted C₆ to C₁₄ aryl groups; and
wherein R³ is -CN and R⁴ is selected from the group consisting of H, -CN, -C(O)R⁶, -C(O)OR⁷, -OC(O)R⁸, -C(O)NR⁹R²⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups, wherein R⁶ to R¹³ are independently selected from the group consisting of H, substituted or unsubstituted C₁ to C₆ alkyl and substituted or unsubstituted C₆ to C₁₄ aryl groups,
or R⁴ is -CN and R³ is selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups, wherein R⁶ to R¹³ are independently selected from the group consisting of H, substituted or unsubstituted C₁ to C₆ alkyl and substituted or unsubstituted C₆ to C₁₄ aryl groups; and
wherein X is C-R⁵, wherein R⁵ is selected from the group consisting of H, substituted or unsubstituted C₁ to C₆ alkyl groups and substituted or unsubstituted C₆ to C₁₄ aryl groups; and
wherein the dashed line represents a double bond;
or a salt of said compound according to formula (I).

These compounds represent a new class of chemical compounds and represent a subgroup of the compounds defined with respect to the pharmaceutical composition. In the following, the new class of compounds being suitable according the present invention will be described in more detail. Especially suitable or preferred subgroups of said compounds will be defined.

According to one embodiment of the present invention, the compound according to formula (I) is further specified in that
R¹ is Cl and R² is selected from the group consisting of methyl and Cl, and preferably is Cl; and
wherein R³ is -CN and R⁴ is selected from the group consisting of H, -CN and -C(O)NR⁹R¹⁰, wherein R⁹ and R¹⁰ are both H, and preferably is -CN
or R⁴ is -CN and R³ is selected from the group consisting of H, -CN and -C(O)NR⁹R¹⁰, wherein R⁹ and R¹⁰ are both H, and preferably is -CN; and
wherein X is C-R⁵, wherein R⁵ is selected from the group consisting of H, methyl, ethyl, n-propyl, trifluoromethyl and phenyl, more preferably is H or methyl, and most preferably is methyl; and
wherein the dashed line represents a double bond;
or a salt of said compound according to formula (I).

According to yet another embodiment of the present invention, the compound according to formula (I) is further specified in that
R¹ is Cl and R² is selected from the group consisting of methyl and Cl, and preferably is Cl; and
wherein R³ is -CN and R⁴ is selected from the group consisting of -CN, and -C(O)NR⁹R¹⁰, wherein R⁹ and R¹⁰ are both H, and preferably is -CN,
or R⁴ is -CN and R³ is selected from the group consisting of -CN and -C(O)NR⁹R¹⁰, wherein R⁹ and R¹⁰ are both H, and preferably is -CN; and
wherein X is C-R⁵, wherein R⁵ is H or methyl, and most preferably is methyl; and
wherein the dashed line represents a double bond;
or a salt of said compound according to formula (I).

Preferably, the inventive compound according to formula (I) is further specified in that
R¹ and R² are both Cl; and
wherein R³ and R⁴ are both -CN; and
wherein X is C-R⁵, wherein R⁵ is selected from the group consisting of H, methyl, ethyl, n-propyl, trifluoromethyl and phenyl, more preferably is H or methyl, and most preferably is methyl; and
wherein the dashed line represents a double bond;
or a salt of said compound according to formula (I).

According to another preferred embodiment of the present invention, the compound is selected from the general formula (II) 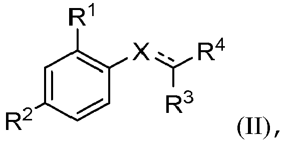 wherein
R¹ is Cl and R² is selected from the group consisting of halogen atoms, substituted or unsubstituted C₁ to C₆ alkyl groups and substituted or unsubstituted C₆ to C₁₄ aryl groups; and
wherein R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups,
or R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups; and
wherein X is C-R⁵, wherein R⁵ is selected from the group consisting of H, substituted or unsubstituted C₁ to C₆ alkyl groups and substituted or unsubstituted C₆ to C₁₄ aryl groups; and
wherein the dashed line represents a double bond;
or a salt of said compound according to formula (II).

The new class of compounds according to formula (II) preferably is further specified in that
R¹ is Cl and R² is selected from the group consisting of halogen atoms or unsubstituted C₁ to C₆ alkyl groups, preferably is Cl or methyl, and most preferably is Cl; and
wherein R³ is -CN and R⁴ is selected from the group consisting of H, methyl, -CN, 1-tetrazole, -C(O)NR⁹R¹⁰, wherein R⁹ and R¹⁰ are both H, preferably R⁴ is selected from the group consisting of H, -CN and -C(O)NR⁹R¹⁰, and most preferably R⁴ is -CN,
or R⁴ is -CN and R³ is selected from the group consisting of H, methyl, -CN, 1-tetrazole, -C(O)NR⁹R¹⁰, wherein R⁹ and R¹⁰ are both H, preferably R³ is selected from the group consisting of H, -CN and -C(O)NR⁹R¹⁰, and most preferably R³ is -CN; and
wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups.

According to another embodiment of the present invention, the compound according to formula (II) is further specified in that
R¹ is Cl and R² is Cl or methyl, and most preferably is Cl; and
wherein R³ is -CN and R⁴ is selected from the group consisting of H, -CN, -C(O)NR⁹R¹⁰, wherein R⁹ and R¹⁰ are both H, preferably R⁴ is selected from the group consisting, and most preferably R⁴ is -CN,
or R⁴ is -CN and R³ is selected from the group consisting of H, -CN, -C(O)NR⁹R¹⁰, wherein R⁹ and R¹⁰ are both H, and most preferably R³ is -CN; and
wherein R⁵ is selected from the group consisting of H, methyl, ethyl, n-propyl, trifluoromethyl and phenyl, and preferably is H or methyl, and most preferably is methyl.

According to one preferred embodiment of the present invention, the compound according to formula (II) is further specified in that
R¹ and R² are both Cl; and
wherein R³ and R⁴ are both -CN; and
wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups.

According to yet another embodiment of the present invention, the compound according to formula (II) is further specified in that
R¹ and R² are both Cl; and
wherein R³ and R⁴ are both -CN; and
wherein R⁵ is selected from the group consisting of H, methyl, ethyl, n-propyl, trifluoromethyl and phenyl, and preferably is H or methyl, and most preferably is methyl.

According to a preferred embodiment of the present invention, the compound is

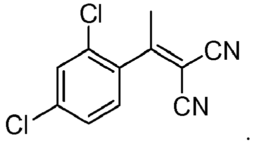

### Synthetic procedures for making the compounds according to the invention

The compounds according to the invention are accessible by synthetic organic chemistry. The person skilled in the art will adjust the synthetic route according to the desired product of the general formula (I). The following general synthetic procedures may be specifically suited for certain molecular subgroups of the general formula (I). However, the scope of the subgroups shall not be understood as being of limiting character. It is also obvious to a skilled person that further synthetic methodology exists for the synthesis of the compounds according to the formula (I). It is also understood by a person skilled in the art that the general synthetic procedures as described herein are not limited to the exemplified and/or described reaction scope. If necessary, a person skilled in the art will modify the general procedures and/or exemplified procedures in order to optimize a reaction and/or to access a compound as defined in formula (I). Unless indicated otherwise, the substituents R¹ to R⁵ and X are defined as specified in the embodiments of the above sections. A "wobbled" bond indicates that the structure encompasses both E and Z diastereomers of the compound and/or that the compound is a mixture of both E and Z diastereomers, unless otherwise indicated.

One synthetic method for obtaining a compound of the general formula (I) is based on a palladium-catalyzed cross coupling reaction between boronic acid A and alkenyl halide B and leads to compounds of general formula C (Suzuki-Miyaura cross coupling, Scheme 1). Substituent Y represents a halide. The reaction may proceed in the presence of 0.0001 mol% to 20 mol% of palladium catalyst such as Pd(PPh₃)₄, based on the amount of halide B. Furthermore, 0.25 equiv. to 5.0 equiv. of a base, with respect to boronic acid A, may be used. The reaction may proceed in a solvent, preferably an organic solvent. The reaction may be carried out at reaction temperatures of from -78°C to 200°C depending on the reactivity of the substrates/catalysts, the solvent used, the solubility etc. Preferably, the reaction is carried out at temperatures above 30°C. Preferably, R⁵ of alkene B is hydrogen and halide Y is a bromide.

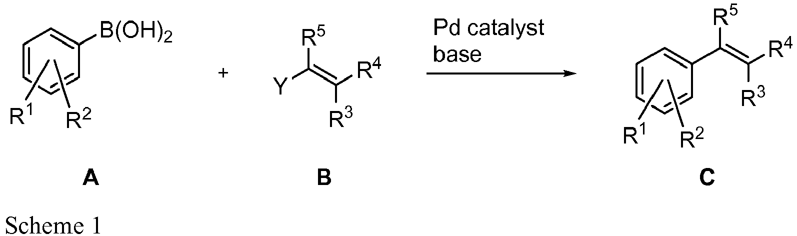

For example, the palladium-catalyzed cross coupling reaction may be carried out under the reaction conditions shown in Scheme 2 and Table 1. In particular, compounds **6, 7** and **18** may be prepared through Suzuki-Miyaura cross-coupling starting from the corresponding disubstituted phenylboronic acid and a mixture of *E*/*Z* 3-bromo-2-methylacrylonirile in the presence of catalytic amounts of Pd(PPh₃)₄ and excess of Cs₂CO₃ as base. The compounds may be obtained as a mixture of their *E:Z* isomers or may be obtained as the pure *E* or *Z* diastereomer after purification by standard purification technology such as silica gel chromatography.

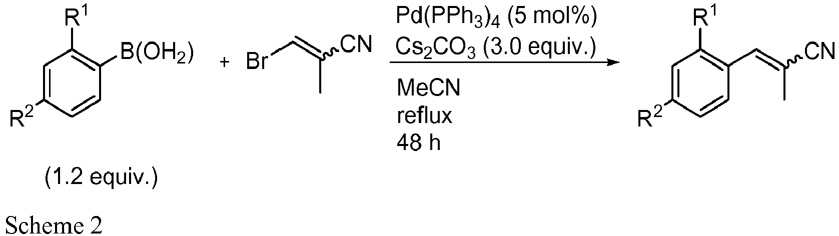

**Table 1:**

| Entry | Product(s) | R¹ | R² | E:Z ratio | Yield [%] |
|---|---|---|---|---|---|
| 1 | *E*-**6** | Cl | Me | >95:5 | 38 |
| 2 | *E*-**7** | Cl | Cl | >95:5 | 31 |
| 3 | *Z*-**6** | Cl | Me | <5:95 | 27 |
| 4 | **6** (*E*:*Z* mixture) | Cl | Me | 51:49 | 75 |
| 5 | *Z*-**7** | Cl | Cl | <5:95 | 46 |
| 6 | **18** (*E*:*Z* mixture) | Me | Me | 52:48 | 88 |

Another synthesis of a compound as described herein, preferably compounds of the general formula F, is based on nucleophilic displacement of secondary halides E with phenol derivatives D (Scheme 3). The reaction is carried out in the presence of a base, preferably 0.25 equiv. to 5.0 equiv. of a base, on the basis of the amount of halide E. The reaction may be carried out in a solvent, preferably an organic solvent. The reaction may be conducted at reaction temperatures of from -78°C to 200°C depending on the reactivity of the substrates, the solvent used, the solubility etc. Preferably, the reaction is carried out at temperatures above 30°C. Preferably, the halide Y is bromide.

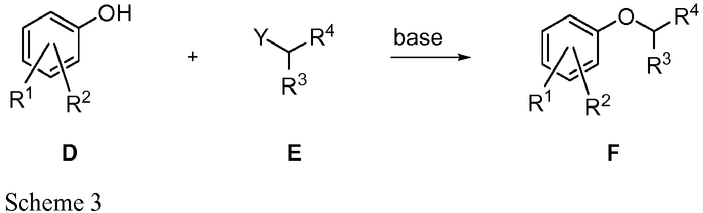

For example, the reaction may proceed under the reaction conditions shown in Scheme 4 and Table 2. Compounds **12** to **15** are obtained in chemical yields above 80% (Table 2).

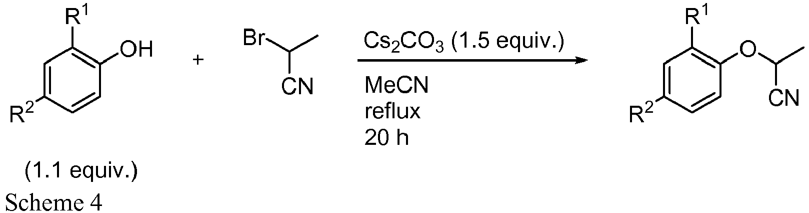

**Table 2:**

| Entry | Compound | R¹ | R² | Yield [%] |
|---|---|---|---|---|
| 1 | **12** | Cl | Cl | 84 |
| 2 | **13** | Cl | Me | 88 |
| 3 | **14** | Me | Me | 94 |
| 4 | **15** | Me | Cl | 86 |

Yet another possibility to synthesize compounds as described herein, preferably compounds of the general formula I, is by condensation of compounds according to formula H with compounds of formula G (Scheme 5). Compounds of formula H may be used in an excess of 1.1 equiv. to 5.0 equiv., based on the amount of compound G. Preferably, compound H is malonic acid, malonic acid ester, or malononitrile, and more preferably malononitrile. R⁵ is preferably hydrogen or an unsubstituted C₁ to C₆ alkyl groups, more preferably a methyl group. The reaction is carried out under acidic conditions in the presence of 0.25 equiv. to 5.0 equiv. of a secondary amine, based on the amount of compound G. The reaction may be carried out in a solvent, preferably a protic solvent such as acidic acid, water, or a mixture thereof. Preferably, the reaction is carried out at temperatures above 30°C. The reaction conditions and/or substrates may correspond to or may be based on the reaction conditions and/or substrates commonly known as Knoevenagel condensation.

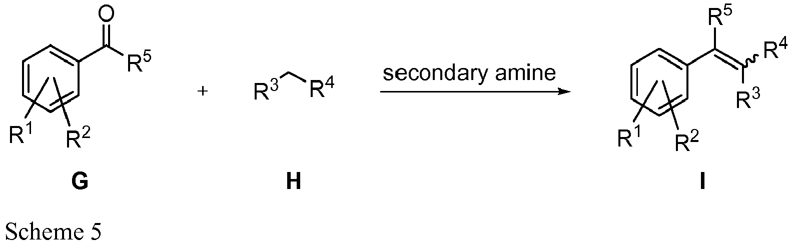

For example, the reaction conditions may be selected as shown in Scheme 6. Compound **1** is synthesized in an excellent yield starting from simple bulk chemicals dichloroacetophenone and malononitrile.

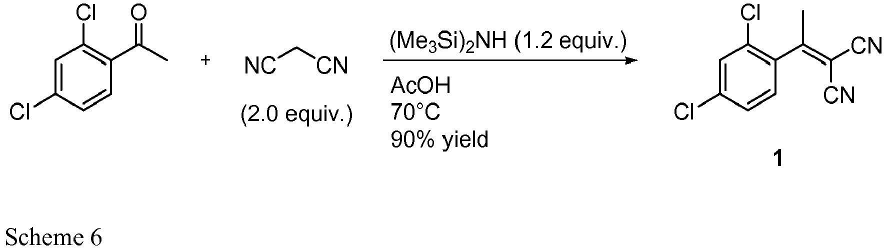

Yet another possibility to synthesize compounds as described herein, preferably compounds of the general formula I, is via Horner-Wadsworth-Evans-type reaction of compounds according to formula G with phosphonates of formula J (Scheme 7). Phosphonates of formula J may be used in an excess of 1.1 equiv. to 5.0 equiv., based on the amount of compound G. R³ and R⁴ of phosphonate I are preferably selected from the group of hydrogen, -CN, -C(O)R⁶, -C(O)OR⁷, -OC(O)R⁸, and more preferably are hydrogen and -CN. R⁵ of compound G is preferably hydrogen or an unsubstituted C₁ to C₆ alkyl groups, and more preferably a methyl group. The reaction may be carried out in the presence of 0.25 equiv. to 5.0 equiv. of a base, based on the amount of compound G. Preferably, the base is a strong base with a pKa of the conjugated acid of at least 15, more preferably of at least 20. The reaction may be carried out in a solvent, preferably an organic solvent. Preferably, the reaction is carried out at temperatures below 30°C.

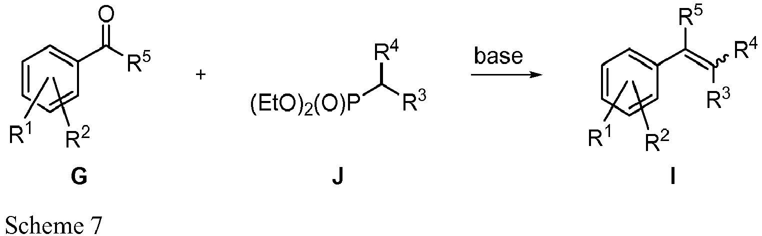

For example, the reaction conditions may be selected as shown in Scheme 8. β,β-disubstituted acrylonitriles **2** and **4** may be assembled through Horner-Wadsworth-Emmons-type reactions (Wadsworth and Emmons, 1961) starting from diethyl cyanomethylphosphonate and acetophenone derivatives in the presence of a lithium amide base (LiHDMS). Chromatographic purification of the thus-obtained compounds may yield pure *E* or *Z* product isomers (Table 3).

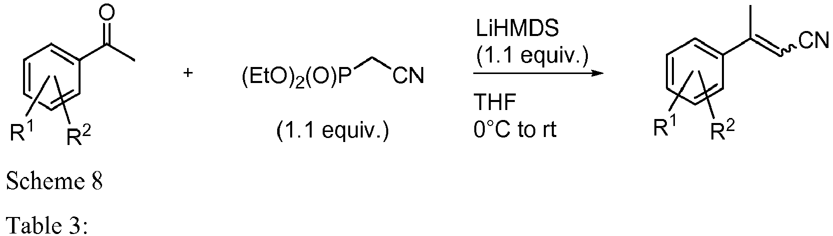

| Entry | Compound(s) | R¹ | R² | E:Z ratio | Yield [%] |
|---|---|---|---|---|---|
| 1 | *Z*-**2** | Cl | Cl | <5:95 | 26 |
| 2 | **4** (*E*:*Z* mixture) | Cl | Me | 60:40 | 80 |
| 3 | *E*-**2** | Cl | Cl | >95:5 | 57 |

Another option for synthesizing compounds as described herein, preferably compounds of the general formula L, is via hydration of nitriles according to the general formula K (Scheme 9). R³ or R⁴ is preferably a -CN group or hydrogen. The assignment of R³ or R⁴ depends on the *Z* or *E* position of the drawn -CN group. The reaction proceeds under acidic conditions. For example, the reaction may be catalyzed/mediated by a Lewis acid or transition metal salt such as a copper(II) salt. The Lewis acid or the transition metal salt may be present in an amount of 0.05 equiv. to 2.0 equiv., based on the amount of the nitrile according to formula K. The reaction may be carried out in a solvent, preferably a protic solvent such as acidic acid, water, or a mixture thereof. Preferably, the reaction is carried out at temperatures above 30°C.

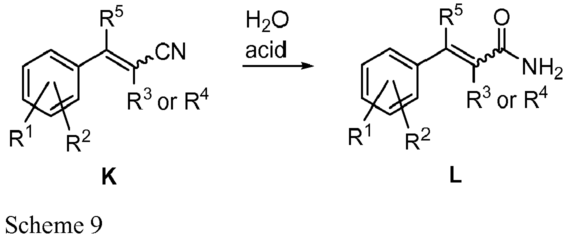

For example, the reaction conditions may be selected as shown in Scheme 10 and Table 4 (Xin et al., 2013). Copper (II)-catalyzed hydration of nitriles afford amide **3** (Scheme 10a) or amide **11** (Scheme 10b). Isomeric *E*:*Z* mixtures may be purified by common purification protocols e.g. based on silica gel chromatography.

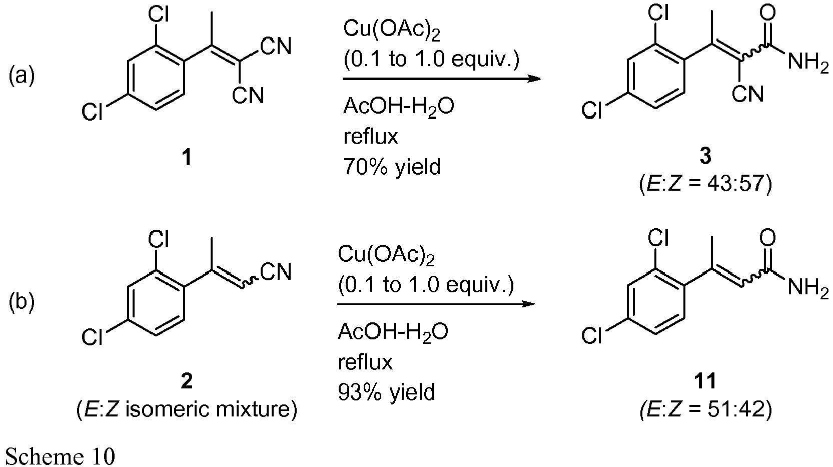

**Table 4:**

| Entry | Compound(s) | E:Z ratio | Yield [%] |
|---|---|---|---|
| 1 | *E*-**3** | >95:5 | 26 |
| 2 | **3** (*E*:*Z* mixture) | 60:40 | 80 |
| 3 | *Z*-**3** | <5:95 | 57 |
| 4 | *E*-**11** | >95:5 | 44 |
| 5 | **11** (*E*:*Z* mixture) | 52:48 | 85 |

Yet another option for synthesizing compounds as described herein, preferably compounds of the general formula M, is via reduction of alkenes according to the general formula I (Scheme 11). The reduction may be carried out with metals such as alkali metals or alkaline earth metals as reducing agents in a protic solvent. Preferably, either R³ or R⁴ is an electron-withdrawing group selected from the group consisting of -CN, -C(O)R⁶, -C(O)OR⁷, -OC(O)R⁸, -C(O)NR⁹R¹⁰, and more preferably CN. Preferably, the reaction is carried out at temperatures below 30°C, for example, at approximately 0°C.

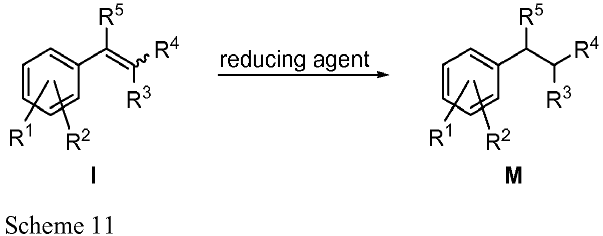

For example, the reaction conditions and reactants may be selected as shown in Scheme 12 and Table 5 (Seebach and Corey, 1975). Reduction with magnesium in methanol provides nitriles **5**, **8** and **9** in excellent yields.

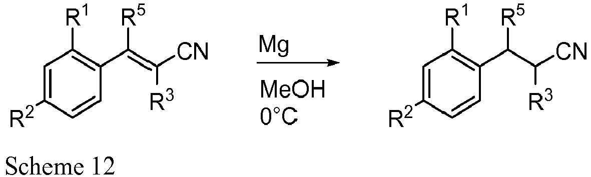

**Table 5:**

| Entry | Compound(s) | R¹ | R² | R³ | R⁵ | Yield [%] |
|---|---|---|---|---|---|---|
| 1 | **5** | Cl | Cl | H | Me | 93 |
| 2 | **8** | Cl | Me | Me | H | 95 |
| 3 | **9** | Cl | Cl | Me | H | 96 |

Yet another option for synthesizing compounds as described herein, preferably compounds of the general formula M, is via reduction/deoxygenation of alcohols according to the general formula N (Scheme 13). The reduction/deoxygenation is carried out with reducing agents, preferably hydride donors such as silanes, in the presence of a Lewis acid. The Lewis acid may be present in an amount of 0.05 equiv. to 1.0 equiv., based on the amount of the alcohol according to formula N. Preferably, the reaction is carried out at temperatures below 30°C, for example, at 0°C or less.

The reaction may be carried out in a solvent, preferably an organic solvent such as dichloromethane.

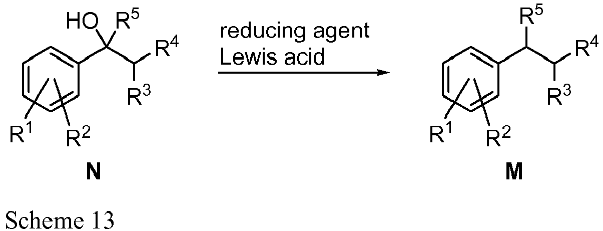

Scheme 14 shows an example for a reduction/deoxygenation reaction of tertiary alcohol. The tertiary alcohol (Mamuye et al., 2015) is reduced to nitrile **10** in the presence of chlorodiphenylsilane as hydride donor and indium chloride as Lewis acid (Yasuda et al., 2001).

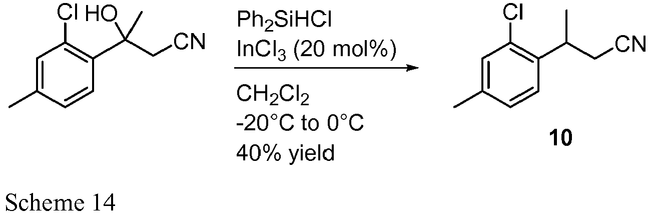

Yet another option for synthesizing compounds as described herein, preferably compounds of the general formula P, is via 1,3-dipolar cycloaddition of nitriles according to the general formula O and azide from an azide source (Scheme 15). The azide source may be, for example, sodium azide or diethylaluminum azide and may be used in excess, i.e. above 2.0 equiv. based on the amount of nitrile O. Preferably, the reaction is carried out at temperatures above 30°C, for example, at approximately 110°C. The reaction may be carried out in a solvent, preferably an organic solvent such as toluene.

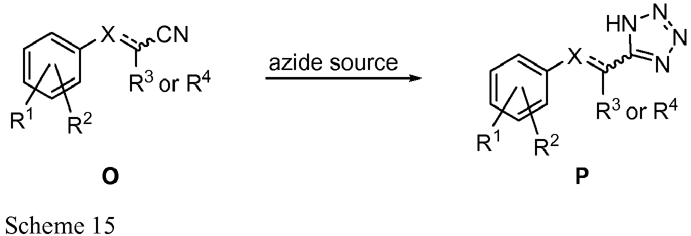

Exemplary 1,3-dipolar cycloadditions are shown in Scheme 16. *E*:*Z* mixtures of acryl nitrile **18** react in the presence of diethylaluminum azide to the corresponding tetrazole **16** (Scheme 16a, Aureggi and Sedelmeier, 2007; Monticelli and Pace, 2015). Cyanohydrine-derived starting material **12** is converted under the same reaction conditions into tetrazole **17** in very good yield (Scheme 16b).

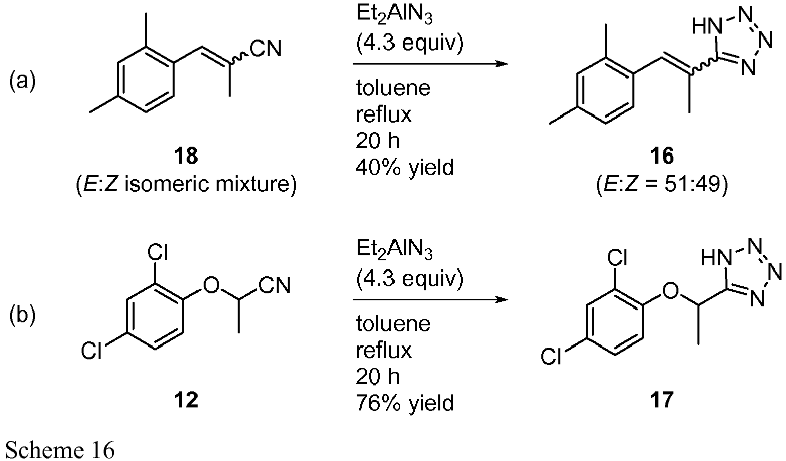

In general, compounds according to general formula (I) having a chiral carbon center may be synthesized in enantiomerically pure form by different methods. Enantiomerically pure compounds may be obtained, for example, by asymmetric synthesis, enantioselective catalysis, chemical and/or chromatographic resolution of racemic compounds and/or by synthetic strategies based on optical pure natural products as starting materials.

Non-limiting examples of enantioselective syntheses of compounds falling under formula (I) are shown in Scheme 17. An enantiomerically pure alpha-chiral amide converts into nitrile (*R*)-**12** without loss of stereoinformation (Scheme 17a). The reaction proceeds in the presence of a mixture of dimethylformamide and oxalyl chloride in THF at room temperature. Alternatively, an enantiomerically pure alpha-chiral ester is transformed by dimethylaluminum amide into the corresponding enantiomerically enriched nitrile (*R*)-**15** in one step.

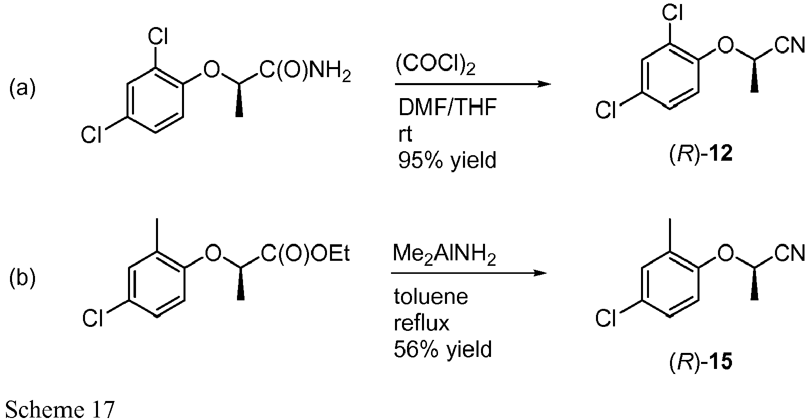

### Formulation and use of the pharmaceutical composition

The compounds according to the present invention or their pharmaceutically acceptable complexes, salts, solvates and hydrates, will be tested by a person skilled in the art for their biopharmaceutical properties, such as solubility and solution stability across pH, permeability, and the like. Based on the result of such tests, the skilled person will select an appropriate dosage form and route of administration for the pharmaceutical composition of the present invention.

In general, the pharmaceutical composition will comprise one or more pharmaceutically acceptable excipients depending in the dosage form and route of administration. Thus, according to one embodiment, the pharmaceutical composition as described herein further comprises one or more pharmaceutically acceptable excipients. Pharmaceutically acceptable excipients are known to the person skilled in the art and include, for example, coating materials, film-forming materials, fillers, disintegrating agents, release-modifying materials, carrier materials, diluents, binding agents, lubricants, emulsifiers, and other adjuvants.

For example, the inventive pharmaceutical composition may be administered orally. Oral administration may involve swallowing in which case the compound enters the bloodstream via the gastrointestinal tract. Alternatively or additionally, oral administration may involve mucosal administration (e.g., buccal, sublingual, supralingual administration) such that the compound enters the bloodstream through the oral mucosa. Formulations suitable for oral administration include solid, semisolid and liquid systems such as tablets; soft or hard capsules containing multi- or nano-particulates, liquids, or powders; films; ovules; sprays; and buccal or mucoadhesive patches. Liquid formulations include suspensions, solutions, syrups and elixirs. Such formulations may be employed as fillers in soft or hard capsules and typically comprise a carrier such as water or methylcellulose and one or more emulsifying agents, suspending agents or both.

If the inventive pharmaceutical composition is administered orally, it may be formulated in form of a tablet dosage form. Tablets may include excipients such as one or more disintegrants, binders, diluents, surfactants, glidants, lubricants, antioxidants, flavoring agents and preservatives. Examples of disintegrants include sodium starch glycolate and sodium carboxymethyl cellulose. Binders are generally used to impart cohesive qualities to a tablet formulation. Suitable binders include, but are not limited to, microcrystalline cellulose, gelatin, sugars, polyethylene glycol and polyvinylpyrrolidone. Tablets may also contain diluents, such as mannitol, xylitol, dextrose, sucrose or sorbitol and surface active agents, such as sodium lauryl sulfate, and further excipients known in the art. Tablet blends may be compressed directly or by roller compaction to form tablets. Tablet blends or portions of blends may alternatively be wet-, dry-, or melt-granulated, melt congealed, or extruded before tableting. If desired, prior to blending one or more of the components may be sized by screening or milling or both. Exemplary tablets may contain up to about 80 wt% of the compound as described herein, from about 10 wt% to about 90 wt% of binder, from about 0 wt% to about 85 wt% of diluent, from about 2 wt% to about 10 wt% of disintegrant, and from about 0.25 wt% to about 10 wt% of lubricant.

In case that liquid dosage forms are considered for the present invention, these can include pharmaceutically acceptable emulsions, solutions, suspensions and syrups containing inert diluents commonly used in the art such as water. These dosage forms may contain e.g. microcrystalline cellulose for imparting bulk, alginic acid or sodium alginate as a suspending agent, methylcellulose as a viscosity enhancer and sweeteners/flavouring agents. When administered by nasal aerosol or inhalation, the compositions according to the present invention may be prepared as solutions in a saline, employing benzyl alcohol or other suitable preservatives, absorption promoters to enhance bioavailability fluorocarbons and/or other solubilising or dispersing agents.

The oral dosage forms may be formulated to ensure an immediate release, a controlled release, or a sustained release of the compound as described herein and/or its pharmaceutically acceptable salt and/or solvate. A "controlled release" dosage form may designate a pharmaceutical dosage form that releases the active agent only after the dosage form has reached a certain site of the body, i.e. the stomach or the gastro-intestinal tract. The term "sustained release" refers to the release of the pharmaceutically active compounds from the dosage form over an extended period of time but not necessarily to the release at a defined place. The term "solvate" describes a molecular complex comprising the compound as described herein and one or more pharmaceutically acceptable solvent molecules (e.g., EtOH). The term "hydrate" refers to a specific solvate in which the solvent is water.

The administration of the pharmaceutical composition is not limited to oral administration and other routes of administration will be chosen by the skilled person, if necessary. For example, the pharmaceutical composition may be administered parentally into the blood stream, muscle, or an internal organ of the subject, topically, intradermally, or transdermally to the skin or mucosa, intranasally or by inhalation, rectally or vaginally, or directly to the eye or ear.

The pharmaceutical compositions in accordance with the present invention may not only comprise the compound as described herein and/or its pharmaceutically acceptable salt and/or solvate but also another pharmaceutically active agents and preferably one or more pharmaceutically active agents which are known to have a positive effect on the treatment and/or prevention of e.g. insomnia, anxiety or epilepsy, when administered to a patient in need of treatment thereof.

For administration to human patients, the total daily dose of the compound as described herein compounds is typically in the range of about 0.1 mg to about 3000 mg depending on the route of administration. For example, oral administration may require a total daily dose of from about 1 mg to about 3000 mg, while an intravenous dose may only require a total daily dose of from about 0.1 mg to about 300 mg. The total daily dose may be administered in single or divided doses and, at the physician's discretion, may fall outside of the typical ranges given above. Although these dosages are based on an average human subject having a mass of about 60 kg to about 70 kg, the physician will be able to determine the appropriate dose for a patient (e.g., an infant) whose mass falls outside of this weight range.

Another suitable criterion for selecting an appropriate amount of the compound as described herein is that the compound and/or pharmaceutically acceptable salt and/or solvate thereof may be administered to an individual in an amount of about 1 to about 100 mg/kg/d, preferably in an amount of about 5 to about 50 mg/kg/d, more preferably in an amount of about 10 to about 25 mg/kg/d and in particular in an amount of about 12 to about 15 mg/kg/d.

In general, a skilled person will be able to select a formulation for the compounds as described herein that ensures a stable blood level and long-lasting action after administration. Thereby, a once daily regimen may be enabled, which is usually particularly desirable for the patient.

As described above, it was surprisingly found that the new class of active agents according to formula (I) and formula (II) were found to provide a modulation of the GABA_{A} receptor response rendering the corresponding pharmaceutical compositions suitable for use as a medicament, e.g. for the treatment of conditions like insomnia, anxiety, cardiac disease and/or epilepsy.

In order to be used for the treatment of conditions like insomnia, anxiety, cardiac disease and/or epilepsy, it may be necessary that the compound as described herein is available in the central nervous system. Thus, it may be advantageous if the compound as described herein is able to penetrate the blood brain barrier. A skilled person may adjust the hydrophobicity of the compound by selecting the appropriate substituents as specified herein, in such a way that the compound may diffuse and/or may be transported from the circulating blood in the brain into the extracellular fluid of the central nervous system.

### FIGURES

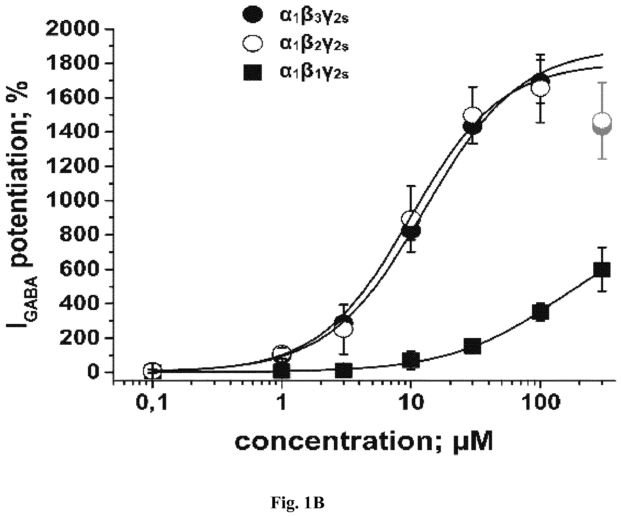

Figure 1
   A) Concentration-dependent I_{GABA} enhancement through α₁β₃γ_{2S} receptors expressed in *Xenopus laevis* oocytes by compound *Z*-**2** (VP-13) compared to prior art agent loreclezole (LOR) and valeric acid (VA).
   B) Compound *Z***-2** (VP-13) enhances I_{GABA} selectively through α₁β₂γ_{2S} and α₁β₃γ_{2S} receptors.
   C) Effect of point mutations resulting in change of amino acid S290 of subunit β1 or N265 of subunit β3, respectively, on the I_{GABA} modulatory effect of compound *Z***-2** (VP-13) (dashed lines represent effects on β₁ and β₃ containing wild type receptor from Fig 1B.
   D) Changes in seizure-threshold upon PTZ-infusion of compound *Z***-2** (VP-13) (squares) and VA (circles; data from Hintersteiner et al., 2014) compared to control animals (dotted line). Compound *Z***-2** (VP-13) elevates the PTZ-induced seizure threshold more potently. Each data point represents the mean±S.E.M from at least 3 mice; (**) indicates statistically significant differences with P<0.01 to control animals.
   E) Effect of point mutations resulting in a change of amino acid F289 of subunit β₃ on the I_{GABA} modulatory effect of compound *Z***-2** (VP-13) (dashed lines represent effects on β₁ and β₃ containing wild type receptor from Fig 1B).

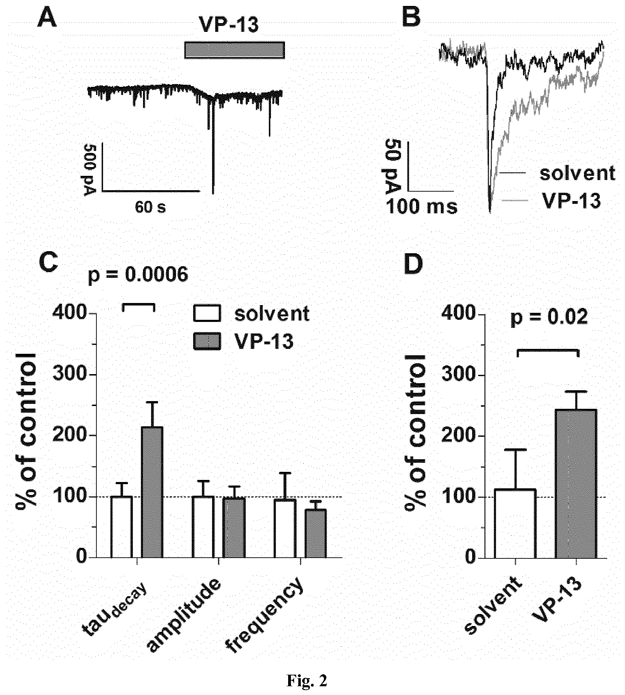

Figure 2 Effects of compound *Z*-**2** (VP-13) on miniature IPSCs and tonic currents in cultured hippocampal neurons. Spontaneously occurring currents were recorded at a holding potential of -70 mV in 0.5 µM TTX plus 10 µM CNQX (control); 10 µM compound *Z*-**2** (VP-13) or 3 µM midazolam (not shown) were applied as indicated. A) and D) Original traces of spontaneous currents at -70 mV in the presence of solvent followed by 10µM compound *Z*-**2** (VP-13). Pronounced effect on the holding current. D) shows statistical analysis. B) Original mIPSCS in the presence of solvent and compound 2 (VP-13). D) Statistical analysis of the effects of compound *Z*-**2** (VP-13) on the decay, amplitude and frequency of GABAergic mIPSCs. Each column represents the averaged value of five neurons (weighted mean ± SD).
Figure 3
   A) β-subunit-dependent I_{GABA} enhancement of compound *Z*-**2** (VP-13). Concentration-dependent modulation of GABA_{A} receptors composed of α₁β₃γ_{2S} (●), α₁β₂γ_{2S} (■), and α₁β₁γ_{2S} (○) subunits by compound *Z*-**2** (VP-13). Grey data point at 300µM (α₁β₂γ_{2S} and α₁β₃γ_{2S} receptors) was excluded from the fit.
   B) Representative traces for the enhancement of GABA-induced chloride currents by 1-300 µM of compound compound *Z*-**2** (VP-13) (double bar indicates coapplication of GABA and compound).
   C) β-subunit-dependent I_{GABA} enhancement of compound 1. Concentration-dependent modulation of GABA_{A} receptors composed of α₁β₃γ_{2S} (●), α₁β₂γ_{2S} (■), and α₁β₁γ_{2S} (○) subunits by compound 1. Grey data point at 300µM (α₁β₂γ_{2S} and α₁β₃γ_{2S} receptors) was excluded from the fit.
   D) β-subunit-dependent I_{GABA} enhancement of compound **4.** Concentration-dependent modulation of GABA_{A} receptors composed of α₁β₃γ_{2S} (●), α₁β₂γ_{2S} (■), and α₁β₁γ_{2S} (○) subunits by compound **4.**
   E) β-subunit-dependent I_{GABA} enhancement of compound *E*-**3**. Concentration-dependent modulation of GABA_{A} receptors composed of α₁β₃γ_{2S} (●), α₁β₂γ_{2S} (■), and α₁β₁γ_{2S} (○) subunits by compound *E*-**3**.
   F) β-subunit-dependent I_{GABA} enhancement of compound *Z***-3**. Concentration-dependent modulation of GABA_{A} receptors composed of α₁β₃γ_{2S} (●), α₁β₂γ_{2S} (■), and α₁β₁γ_{2S} (○) subunits by compound *Z***-3.**

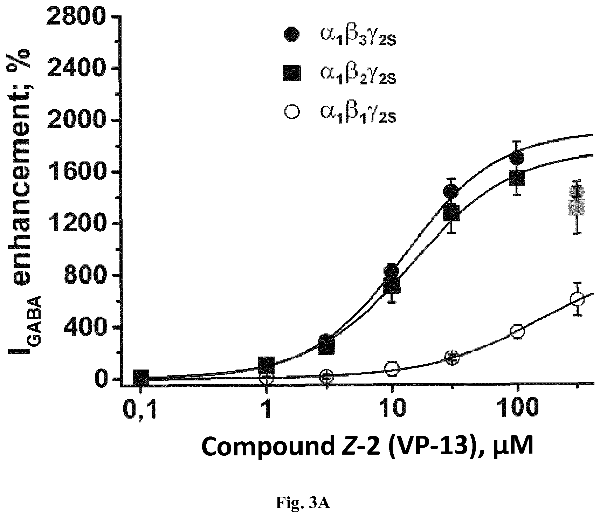

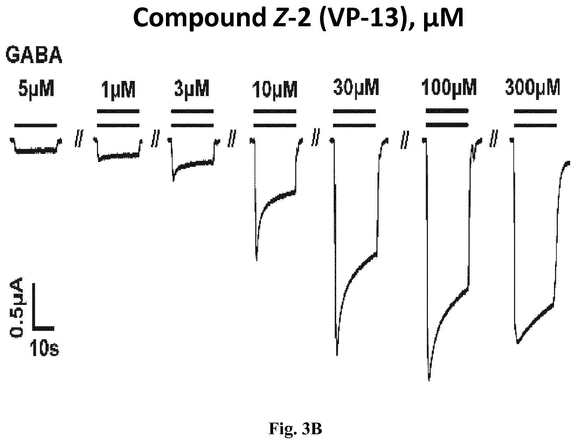

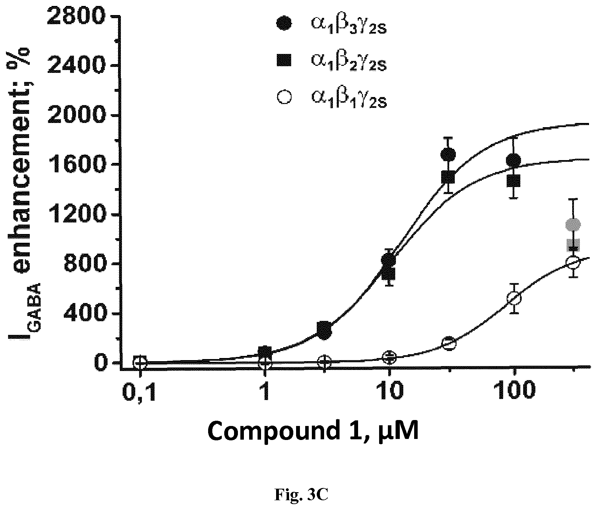

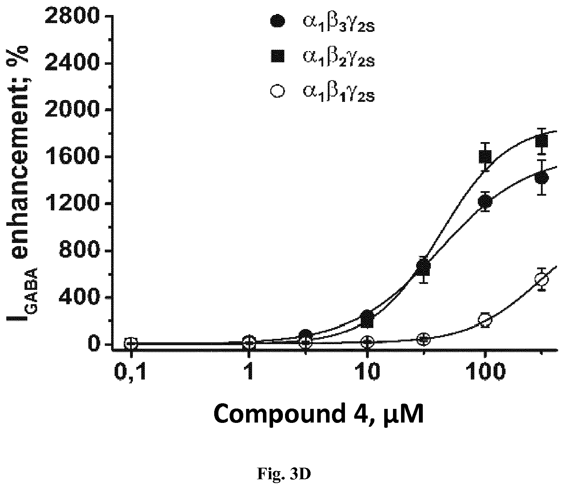

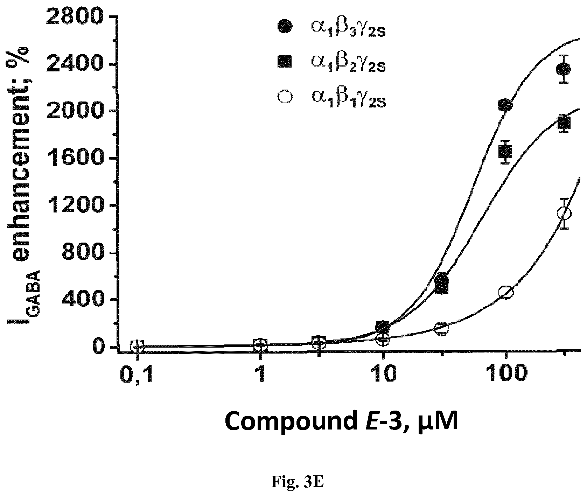

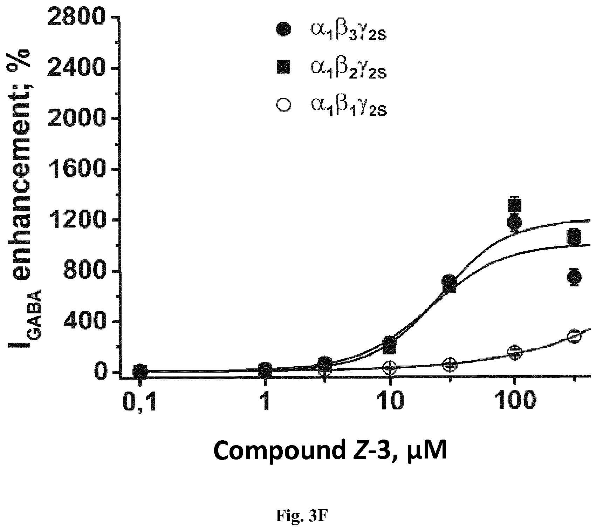

Data in Figure 3A to 3F were fitted by non-linear regression as described in Materials and Methods. Each data point represents a mean ± SEM from at least 3 different oocytes from 2 different frog batches. Error bars smaller than the symbol are not shown.

### EXAMPLES

### 1) Synthesis of compounds

### Instrumentation and General Analytical Methods

Melting points were determined on a Reichert-Kofler hot-stage microscope and are uncorrected. Mass spectra were obtained on a Shimadzu QP 1000 instrument (EI, 70 eV) and on a Bruker maXis 4G instrument (ESI-TOF, HRMS). ¹H, ¹³C, ¹⁹F NMR spectra were recorded with a Bruker Avance III 400 spectrometer (400 MHz for ¹H, 100 MHz for ¹³C, 377 MHz for ¹⁹F), with a Bruker Avance III 500 spectrometer (500 MHz for ¹H, 125 MHz for ¹³C) and with a Bruker DRX spectrometer (200 MHz for ¹H, 50 MHz for ¹³C) at 297 K. The center of the solvent signal was used as an internal standard which was related to TMS with δ 7.26 ppm (¹H in CDCl₃), δ 7.16 ppm (¹H in C₆D₆), δ :5.32 ppm (¹H in CD₂D₂), δ 4.78 ppm (¹H in CD₃OD), δ 77.00 ppm (¹³C in CDCl₃), δ 54.00 ppm (¹³C in CD₂D₂), δ 49.15 ppm (¹³C in CD₃OD) and δ 128.06 ppm (¹³C in C₆D₆). ¹⁹F NMR spectra by absolute referencing via 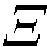 ratio. Spin-spin coupling constants (*J*) are given in Hz. In nearly all cases, full and unambiguous assignment of all resonances was performed by combined application of standard NMR techniques, such as APT, HSQC, HMBC, COSY andNOESY experiments. THF was distilled over Na/benzophenone. Chemicals were purchased from Sigma-Aldrich, Acros, Alfa Aesar, Fluorochem and TCI Europe, otherwise specified. Solutions were evaporated under reduced pressure with a rotary evaporator. TLC was carried out on aluminium sheets precoated with silica gel 60F254 (Macherey-Nagel, Merk); the spots were visualized under UV light (λ = 254 nm) and/or KMnO₄ (aq.) was used as revealing system.

### General synthetic procedures

### General Procedure A (Nucleophilic displacement)

A mixture of 2,4-disubstitutedphenol (1.1 equiv), 2-bromopropionitrile (1.0 equiv) and Cs₂CO₃ (1.5 equiv) in acetonitrile was refluxed for 20 h. After cooling to room temperature, the reaction mixture was quenched with H₂O. The aqueous phase was extracted with DCM, the combined organic phases were washed with brine, dried over Na₂SO₄, filtered and concentrated under reduced pressure. The crude was purified by chromatography on silica gel (hexane/AcOEt) to obtain the pure product. The reaction as described in scheme 4 above may be carried out according to the general procedure A.

### General Procedure B (Suzuki-Miyaura cross-coupling)

A mixture of (disubstituted) phenylboronic acid (1.2 equiv), *cis*/*trans*-3-bromo-2-methylacrylonirile (1.0 equiv), Cs₂CO₃ (3.0 equiv) and bis(triphenylphosphine)palladium(II)dichloride (5 mol%) in acetonitrile was refluxed for 48 h. After cooling to room temperature, the mixture was filtered off with a Büchner funnel containing Celite®. The filtrate was extracted three times with DCM, the organic phases were combined, washed with brine, dried over Na₂SO₄, filtered and concentrated under reduced pressure to afford crude. The residue was purified by chromatography on silica gel (petrol ether/AcOEt) to obtain the pure product. The reaction as described in scheme 2 above may be carried out according to the general procedure B; tetrakis(triphenylphosphine) palladium(0) or bis(triphenylphosphine) palladium(II) dichloride may be used as catalyst.

### General Procedure C (Wittig-Horner-Emmons)

Lithium bis(trimethylsilyl)amide (LHMDS) (1.0 M in THF, 1.1 equiv) was added to a solution of diethyl cyanomethylphosphonate (1.1 equiv) in dry THF under Argon. The solution was stirred for 30 min at room temperature and then was cooled at 0°C. A solution of the ketone (1.0 equiv) in THF was slowly added and stirred for 10 min at 0 °C. The cooling bath was removed and the stirring was continued for 45 min at room temperature. The reaction mixture was quenched with sat. aqueous NH₄Cl solution and stirred 1-2 min before AcOEt was added and the phases separated. The organic layer was washed with water, dried over Na₂SO₄, filtered and concentrated under reduced pressure. The crude was purified by chromatography on silica gel (hexane/AcOEt) to obtain the pure product. The reaction as described in scheme 8 above may be carried out according to the general procedure C.

### General Procedure D (Copper catalyzed hydration of nitriles)

A mixture of nitrile (1.0 equiv) and Cu(OAc)₂ (0.1-1.0 equiv) in HOAc containing H₂O was stirred at given temperature for 24-72 h and then, poured into sat. aqueous NaCl, before to be extracted with DCM. The organic layer was washed with water, dried over Na₂SO₄, filtered and concentrated under reduced pressure. The crude was purified by chromatography on silica gel (hexane/AcOEt) to obtain the pure product. The reaction as described in scheme 10 above may be carried out according to the general procedure D. Further information about the copper-catalyzed hydration of nitriles may be taken from the publication: Xin, X., Xiang, D., Yang, J., Zhang, Q., Zhou, F., Dong, D. J. Org. Chem., 2013, 78, 11956-11961.

### General Procedure E (Reduction of conjugated nitriles with magnesium)

To a solution of nitrile (1.0 equiv) in MeOH was added magnesium turnings (40.0 equiv). The exothermic reaction which ensued after 10 min was moderated with ice bath. The reaction was stirred at 0 °C and then at room temperature overnight. The reaction was quenched with a solution of HCl 6N over 1h period to afford a clear solution which was extracted with Et₂O. The organic layer was washed with water, dried over Na₂SO₄, filtered and concentrated under reduced pressure. The crude was purified by chromatography on silica gel (hexane/AcOEt) to obtain the pure product. The reactions as described in scheme 12 above may be carried out according to the general procedure E. Further information on the reduction of conjugated nitriles may be taken from the publication: Profitt, J. A., Watt, D. S., Corey, E. J., J. Org. Chem., 1975, 40, 127-128.

### General Procedure F (Synthesis of 5-substituted tetrazoles from organoaluminum azides and nitriles)

An oven-dried flask was charged with sodium azide (4.3 equiv) and diethylaluminum chloride (4.3 equiv) at 0 °C under argon and stirred for 15 min. The white, heterogeneous mixture was then warmed to room temperature and stirred for 6 hours. During the formation of the reagent, a suspension of sodium chloride was formed. Nitrile (1.0 equiv) was added at room temperature in two portions. The mixture was gradually heated to 55 °C. When TLC analysis showed complete conversion, the reaction mixture was cooled to 0 °C and added to a solution of NaOH 15% (13.0 equiv) containing sodium nitrite (13.0 equiv, solution pH 13.5). The pH value was adjusted to 1.5 with HCl 6M. The solution was extracted with ethyl acetate to afford the crude product, which was redissolved in ethyl acetate and extracted with aqueous potassium carbonate (10%) to the aqueous phase as the potassium salt (pH 11). The combined basic aqueous phase was cooled to 0 °C and carefully treated with HCl 6M to adjust the pH value to 2.5. The product was extracted with ethyl acetate and concentrated under reduced pressure. The reactions as described in scheme 16 above may be carried out according to the general procedure F. Further information on the synthesis of 5-substituted tetrazoles from organoaluminum azides and nitriles may be taken from the paublication: Aureggi, V., Sedelmeier, G. Angew. Chem. Int. Ed.2007, 46, 8440-8444.

### Synthesis and characterization of compounds

### 2-(1-(2,4-dichlorophenyl)ethylidene)malononitrile (1)

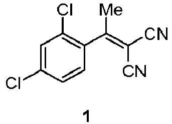

Slowly, 1,1,1,3,3,3-hexamethyl-disilazane (1.025 g, 6.35 mmol, 1.2 equiv) was added to acetic acid (3.54 mL, 61.9 mmol, 11.7 equiv) while keeping the temperature below 75 °C. The mixture was added to a solution of 1-(2,4-dichlorophenyl)ethan-1-one (1.0 g, 5.29 mmol, 1.0 equiv) and malononitrile (0.699 g, 10.58 mmol, 2.0 equiv) in acetic acid (1.81 mL, 31.74 mmol, 6.0 equiv). The reaction mixture was heated at 75 °C for 18 h, after which it was allowed to cool to room temperature. The reaction mixture was diluted with toluene (250 mL), water was added (100 mL), and the acetic acid layer was separated and extracted with AcOEt. The organic layer was washed with water, dried over Na₂SO₄, filtered and concentrated under reduced pressure. The crude was purified by chromatography on silica gel (hexane/AcOEt). Compound 1 was obtained in 90% yield (1.123 g) as transparent oil.
**¹H NMR** (400 MHz, CDCl₃) δ: 7.54 (dd, *J* = 2.0, 0.3 Hz, 1H, Ph H-3), 7.39 (dd, *J* = 8.3, 2.0 Hz, 1H, Ph H-5), 7.16 (dd, *J* = 8.3, 0.3 Hz, 1H, Ph H-6), 2.59 (s, 3H, CH₃). **¹³C NMR** (101 MHz, CDCl₃) δ: 174.2 (CCH₃), 137.6 (Ph C-4), 134.0 (Ph C-1), 131.7 (Ph C-2), 130.7 (Ph C-3), 128.8 (Ph C-6), 128.1 (Ph C-5), 111.2 (C≡N), 111.1 (C≡N), 90.2 (C(C≡N)₂), 24.4 (CH₃).
**HRMS (ESI),** *m*/*z:*calcd. for C₁₁H₆Cl₂N₂Na: 258.9800 [M+Na]⁺; found: 258.9795.

### 3-(2,4-dichlorophenyl)but-2-enenitrile (2)

According to *General Procedure C,* LHMDS 1.0 M in THF (2.91 mL, 2.91 mmol, 1.1 equiv) was added to a solution of diethyl cyanomethylphosphonate (0.515 g, 2.91 mmol, 1.1 equiv) in dry THF (25 mL) under Argon. The solution was stirred for 30 min at room temperature and then was cooled at 0 °C. A solution of freshly prepared 1-(2-chloro-4-methylphenyl)ethan-1-one (0.500 g, 2.64 mmol, 1.0 equiv) in THF (3 mL) was slowly added. The residue was purified by chromatography on silica gel (petrol ether/AcOEt; 95:5). 3-(2,4-Dichlorophenyl)but-2-enenitrile was obtained in 83% yield (0.462 g) as mixture of (*E*)-**2** (0.318g) as a white solid; mp35-39 °C and (*Z*)-**2** (0.144g); *E*:*Z* ratio =69:31.

### (2E)-3-(2,4-dichlorophenyl)but-2-enenitrile (E-2)

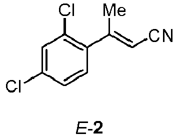

**¹H NMR** (400 MHz, CDCl₃) δ: 7.44 (d, *J* = 2.0 Hz, 1H, Ph H-3), 7.28 (dd, *J* = 8.3, 2.0 Hz, 1H, Ph H-5), 7.11 (d, *J* = 8.3 Hz, 1H, Ph H-6), 5.36 (q, *J* = 1.2 Hz, 1H, CH), 2.41 (d, *J* = 1.2 Hz, 3H, CH₃).
**¹³C NMR** (101 MHz, CDCl₃) δ: 159.4 (C-C≡N), 137.7 (Ph C-1), 135.5 (Ph C-4), 132.2 (Ph C-2), 130.1 (Ph C-3), 129.6 (Ph C-6), 127.4 (Ph C-5), 116.1 (C≡N), 101.1 (CH), 22.2 (CH₃).
**HRMS (ESI),** *m*/*z:*calcd. for C₁₀H₇Cl₂NNa: 233.9848 [M+Na]⁺; found: 233.9846.

### (2Z)-3-(2,4-dichlorophenyl)but-2-enenitrile (Z-2)

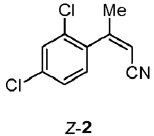

**¹H NMR** (400 MHz, CDCl₃) δ: 7.47 (d, *J* = 2.0 Hz, 1H, Ph H-3), 7.32 (dd, *J* = 8.3, 2.0 Hz, 1H, Ph H-5), 7.17 (d, *J* = 8.3 Hz, 1H, Ph H-6), 5.56 (q, *J* = 1.6 Hz, 1H, CH), 2.24 (d, *J* = 1.6 Hz, 3H, CH₃).
**¹³C NMR** (101 MHz, CDCl₃) δ: 159.9 (CCH₃), 136.3 (Ph C-1), 135.5 (Ph C-4), 132.0 (Ph C-2), 130.1 (Ph C-3), 129.6 (Ph C-6), 127.6 (Ph C-5), 115.9 (C≡N), 100.4 (CH), 24.4 (CH₃).
**HRMS (ESI),** *m*/*z:*calcd. for C₁₀H₇Cl₂NNa: 233.9848 [M+Na]⁺; found: 233.9845.

### 2-cyano-3-(2,4-dichlorophenyl)-2-butenamide (3)

According to *General Procedure D,* a mixture of **1** (0.200 g, 0.847 mmol, 1.0 equiv) and Cu(OAc)₂(0.015 g, 0.085 mmol, 1.0 equiv), H₂O (0.2 mL) in HOAc (8.5 mL) was stirred at 80 °C for 24 h and then poured into saturated aqueous NaCl, which was extracted with DCM. The crude was purified by chromatography on silica gel (hexane/AcOEt; 80:20). Compound **3** was obtained in 70% yield (0.150 g) as mixture of (*E*)-**3** (0.064 g) as white solid; mp144-147 °C and (*Z*)-**3** (0.086 g) as white solid; mp149-153 °C; *E*:*Z* ratio = 43:57.

### (2E)-2-cyano-3-(2,4-dichlorophenyl)-2-butenamide (E-3)

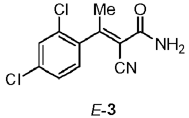

**¹H NMR** (400 MHz, CDCl₃) δ: 7.43 (d, *J* = 2.0 Hz, 1H, Ph H-3), 7.28 (dd, *J* = 8.3, 2.0 Hz, 1H, Ph H-5), 7.00 (d, *J* = 8.3 Hz, 1H, Ph H-6), 5.92 (br s, 1H, NH₂), 5.85 (br s, 1H, NH₂), 2.46 (s, 3H, CH₃).
**¹³C NMR** (101 MHz, CDCl₃) δ: 163.9 (CCH₃), 160.9 (C=O), 136.5 (Ph C-1), 135.2 (Ph C-4), 130.8 (Ph C-2), 129.6 (Ph C-3), 127.9 (Ph C-6), 127.4 (Ph C-5), 115.7 (C≡N), 109.8 (CC=O), 26.0 (CH₃).
**HRMS (ESI),** *m*/*z:*calcd. for C₁₁H₈Cl₂N₂NaO: 276.9894 [M+Na]⁺; found: 276.9896.

### (2Z)-2-cyano-3-(2,4-dichlorophenyl)-2-butenamide (Z-3)

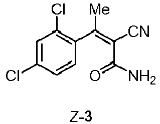

**¹H NMR** (400 MHz, CDCl₃) δ: 7.51 (dd, *J* = 2.0, 0.3 Hz, 1H, Ph H-3), 7.35 (dd, *J* = 8.3, 2.0 Hz, 1H, Ph H-5), 7.15 (dd, *J* = 8.3, 0.3 Hz, Ph H-6), 6.25 (br s, 1H, NH₂), 5.78 (br s, 1H, NH₂), 2.63 (s, 3H, CH₃).
**¹³C NMR** (101 MHz, CDCl₃) δ: 168.2 (C-CH₃), 162.0 (C=O), 138.2 (Ph C-1), 136.1 (Ph C-4), 131.5 (Ph C-2), 130.3 (Ph C-3), 128.8 (Ph C-6), 127.9 (Ph C-5), 116.1 (C≡N), 109.0 (CC=O), 22.5 (CH₃)
**HRMS (ESI),** *m*/*z:*calcd. for C₁₁H₈Cl₂N₂NaO: 276.9894 [M+Na]⁺; found: 276.9896.

### 3-(2-chloro-4-methylphenyl)-2-butenenitrile (4)

According to *General Procedure C,* LHMDS 1.0 M in THF (4.57 mL, 4.57 mmol, 1.1 equiv) was added to a solution of diethyl cyanomethylphosphonate (0.809 g, 4.57 mmol, 1.1 equiv) in dry THF (50 mL) under argon. The solution was stirred for 30 min at room temperature and then was cooled at 0 °C. A solution of freshly prepared 1-(2-chloro-4-methylphenyl)ethan-1-one (0.700 g, 4.15 mmol, 1.0 equiv) in THF (5 mL) was slowly added. The residue was purified by chromatography on silica gel (petrol ether/AcOEt, 95:5). Compound **4** was obtained in 80% yield (0.634 g) as yellow oil; *E*:*Z* ratio = 60:40.

### E-4:

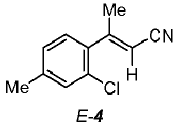

**¹H NMR** (500 MHz, CDCl₃) δ: 7.23 (s, 1H, Ph H-3), 7.08 (d, *J* = 7.85 Hz, 1H, Ph H-5), 7.05 (d, *J* = 7.9 Hz, 1H, Ph H-6), 5.35 (s, 1H, C=CH), 2.41 (s, 3H, CH₃), 2.34 (s, 3H, Ph-CH₃)
**¹³C NMR** (125 MHz, CDCl₃) δ: 160.7 (CCH₃), 140.6 (Ph C-4), 136.3 (Ph C-1), 130.9 (Ph C-2), 130.7 (Ph C-3), 128.6 (Ph C-6), 127.7 (Ph C-5), 116.5 (C≡N), 100.3 (=CH-), 24.7 (CH₃), 20.9 (Ph-CH₃)

### Z-4 :

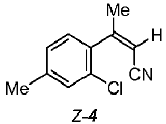

**¹H NMR** (500 MHz, CDCl₃) δ: 7.26 (s, 1H, Ph, H-3), 7.11 (AB system, *J_{AB}* = 9.0 Hz, 2H, Ph H-5,6), 5.51 (s, 1H, C=CH), 2.35 (s, 3H, Ph-CH₃), 2.24 (s, 3H, CH₃)
**¹³C NMR** (125 MHz, CDCl₃) δ: 161.3 (CCH3), 140.6 (Ph C-4), 134.8 (Ph C-1), 131.1 (Ph C-2), 130.6 (Ph C-3), 128.6 (Ph C-6), 127.9 (Ph C-5), 116.3 (C≡N), 99.6 (=CH-), 22.3 (CH₃), 20.9 (Ph-CH₃)
**HRMS (ESI),** *m*/*z:*calcd. for C₁₁H₁₀ClNNa: 214.0394 [M+Na]⁺; found: 214.0395

### 3-(2,4-dichlorophenyl)butanenitrile (5)

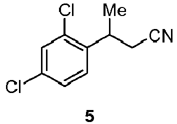

According to *General Procedure E,* to a solution of **2** (0.50 g, 0.236 mmol, 1.0 equiv) in MeOH (1.5 mL) was added magnesium turnings (0.229 g, 9.44 mmol, 40.0 equiv). The exothermic reaction which ensued after 10 min was moderated with ice bath. The reaction was stirred for 1,5 h at 0 °C. The crude was purified by chromatography on silica gel (hexane / AcOEt, 90:10). Compound **5** was obtained in 93% yield (0.047 g) as transparent oil.
**¹H NMR** (400 MHz, CDCl₃) δ: 7.41 (dd, *J* = 1.7, 0.8 Hz, 1H, Ph H-3), 7.27 (m, 1H, Ph H-5), 7.27 (m, 1H, Ph H-6), 3.66 (qd, *J* = 7.0, 5.7 Hz, 1H, CH), 2.67 (dd, *J* = 17.9, 5.7 Hz, 1H, CH₂), 2.58 (dd, *J* = 17.9, 5.7 Hz, 1H, CH₂), 1.46 (d, *J* = 7.0 Hz, 3H, CH₃). **¹³C NMR** (101 MHz, CDCl₃) δ: 138.5 (Ph C-1), 134.1 (Ph C-2), 133.6 (Ph C-4), 129.7 (Ph C-3), 127.9 (Ph C-6), 127.7 (Ph C-5), 117.8 (C=N), 31.8 (CH), 24.6 (CH₂), 19.0 (CH₃).
**HRMS (ESI),** *m*/*z:*calcd. for C₁₀H₉Cl₂NNa 236.0004 [M+Na]⁺; found: 236.0000.

### 3-(2-chloro-4-methylphenyl)-2-methylacrylonitrile (6)

According to *General Procedure B,* a mixture of 2-chloro-4-methylphenylboronic acid (0.284 g, 1.66 mmol, 1.2 equiv), *cis*/*trans*3-bromo-2-methylacrylonirile (0.201 g, 1.38 mmol, 1.0 equiv), Cs₂CO₃(1.349 g, 4.14 mmol, 3.0 equiv) and bis(triphenylphosphine)palladium(II)dichloride (0.048 g, 0.069 mmol, 5 mol%) in acetonitrile (10 mL) was refluxed for 48 h. The residue was purified by chromatography on silica gel (petrol ether/AcOEt, 95:5). Compound **6** was obtained in 75% yield (0.198 g) as mixture of (*E*)-**6** (0.101 g) as white solid; mp116 °C and (*Z*)**-6** (0.097 g) as transparent oil; *E*:*Z* ratio =51:49.

### (2E)-3-(2-chloro-4-methylphenyl)-2-methylacrylonitrile (E-6)

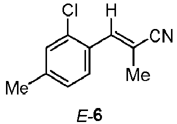

**¹H NMR** (500 MHz, CDCl₃) δ: 7.33 (s, 1H, C=CH), 7.27 (s, 1H, Ph H-3), 7.17 (d, *J* = 7.9 Hz, 1H, Ph H-6), 7.10 (d, *J* = 8.3 Hz, 1H, Ph H-5), 2.36 (s, 3H, Ph-CH₃), 2.05 (d, *J* = 1.6 Hz, 3H, CH₃)
**¹³C NMR** (125 MHz, CDCl₃) δ: 141.4 (=CH-), 141.1 (Ph C-4), 133.8 (Ph C-2), 130.4 (Ph C-3), 129.9 (Ph C-6), 129.2 (Ph C-1), 127.4 (Ph C-5), 120.7 (C≡N), 111.1 (C-C≡N), 21.1 (Ph-CH₃), 16.7 (CH₃)
**HRMS (ESI),** *m*/*z:*calcd. for C₁₁H₁₀ClNNa: 214.0394 [M+Na]⁺; found: 214.0395

### (2Z)-3-(2-chloro-4-methylphenyl)-2-methylacrylonitrile (Z-6)

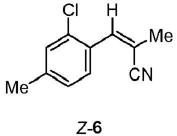

**¹H NMR** (500 MHz, CDCl₃) δ: 7.83 (d, *J* = 8.0 Hz, 1H, Ph H-6), 7.24 (s, 1H, Ph H-3), 7.24 (s, 1H, C=CH), 7.13 (d, *J* = 7.4 Hz, 1H, Ph H-5), 2.35 (s, 3H, Ph-CH₃), 2.18 (d, *J* = 1.6 Hz, 3H, CH₃)
**¹³C NMR** (125 MHz, CDCl₃) δ: 141.4 (Ph C-4), 140.6 (=CH-), 133.5 (Ph C-2), 130.1 (Ph C-3), 129.3 (Ph C-1), 128.7 (Ph C-6), 127.9 (Ph C-5), 118.8 (C≡N), 108.5 (C-C≡N), 21.9 (CH₃), 21.1 (Ph- CH₃)
**HRMS (ESI),** *m*/*z:*calcd. for C₁₁H₁₀ClNNa: 214.0394 [M+Na]⁺; found: 214.0395

### 3-(2,4-dichlorophenyl)-2-methylacrylonitrile (7)

According to *General Procedure B,* a mixture of 2,4-dichlorophenylboronic acid (0.687 g, 3.6 mmol, 1.2 equiv), *cis*/*trans*3-bromo-2-methylacrylonirile (0.438 g, 3.00 mmol, 1.0 equiv), Cs₂CO₃ (2.932 g, 9.0 mmol, 3.0 equiv) and bis(triphenylphosphine)palladium(II)dichloride (0.105 g, 0.15 mmol, 5 mol%) in acetonitrile (30 mL) was refluxed for 48 h. The residue was purified by chromatography on neutral alumina (grade II) (petrol ether/AcOEt, 95:5). 3-(2,4-dichlorophenyl)-2-methylacrylonitrile was obtained in 77% yield (0.487g) as mixture of (*Z*)-**7** (0.282g) as a white solid; mp71-74 °C and (*E*)-**7** (0.205.g) as a white solid; mp 59-62 °C; *E*:*Z* ratio = 42:58

### (2Z)-3-(2,4-dichlorophenyl)-2-methylacrylonitrile (Z-7)

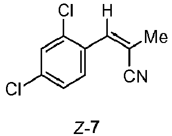

**¹H NMR** (500 MHz, CDCl₃) δ: 7.86 (d, *J* = 8.4 Hz, 1H, Ph H-6), 7.44 (s, 1H, Ph H-3), 7.32 (d, *J* = 8.4 Hz, 1H, Ph H-5), 7.20 (s, 1H, C=CH₃), 2.20 (s, 3H, CH₃).
**¹³C NMR** (125 MHz, CDCl₃) δ: 139.5 (=C-), 136.0 (Ph C-4), 134.4 (Ph C-2), 130.7 (Ph C-1), 129.8 (Ph C-5), 129.6 (Ph C-3), 127.5 (Ph C-6), 118.3 (C≡N), 110.2 (C-C≡N), 22.0(CH₃).
**HRMS (ESI),** *m*/*z:*calcd. for C₁₀H₇Cl₂NNa: 233.9848 [M+Na]⁺; found: 233.9852.

### (2E)-3-(2,4-dichlorophenyl)-2-methylacrylonitrile (E-7)

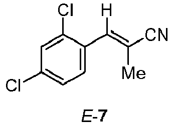

**¹H NMR** (500 MHz, CDCl₃) δ: 7.48 (s, 1H, Ph H-3), 7.30 (m, 1H, Ph H-5), 7.28 (m, 1H, C=CH), 7.22 (d, J = 8.3 Hz, 1H), 2.04 (s, 3H).
**¹³C NMR** (125 MHz, CDCl₃) δ: 140.3 (=C-), 135.7 (Ph C-4), 134.7 (Ph C-2), 130.8 (Ph C-6), 130.6 (Ph C-1), 129.9 (Ph C-3), 127.1 (Ph C-5), 120.2 (C≡N), 112.5 (C-C≡N), 16.7 (CH₃).
**HRMS (ESI),** *m*/*z:*calcd. for C₁₀H₇Cl₂NNa: 233.9848 [M+Na]⁺; found: 233.9850.

### 3-(2-chloro-4-methylphenyl)-2-methylpropanenitrile (8)

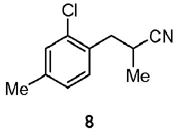

According to *General Procedure E,* to a solution of **6** (0.100 g, 0.52 mmol, 1.0 equiv) in MeOH (2.5 mL) was added magnesium turnings (0.506 g, 20.8 mmol, 40.0 equiv). The exothermic reaction which ensued after 10 min was moderated with ice bath. The reaction was stirred for 1h at 0 °C and then at room temperature overnight. The crude was purified by chromatography on silica gel (hexane / AcOEt, 90:10). Compound **8** was obtained in 95% yield (0.095 g) as transparent oil.
**¹H NMR** (400 MHz, CDCl₃) δ: 7.20 (m, 1H, Ph H-3), 7.19 (m, 1H, Ph H-6), 7.05 (m, 1H, Ph H-5), 2.97 (m, 1H, CH), 2.97 (m, 2H, CH₂), 2.32 (s, 3H, Ph-CH₃), 1.36 (m, 3H, CH₃).
**¹³C NMR** (101 MHz, CDCl₃) δ: 139.1 (Ph C-4), 133.6 (Ph C-2), 131.5 (Ph C-1), 131.2 (Ph C-6), 130.2 (Ph C-3), 127.9 (Ph C-5), 122.4 (C≡N), 37.5 (CH₂), 26.0 (CH), 20.8 (Ph-CH₃), 17.8 (CH₃).
**HRMS (ESI),** *m*/*z:*calcd. for C₁₁H₁₂ClNNa: 216.0550 [M+Na]⁺; found: 216.0553.

### 3-(2,4-dichorophenyl)-2-methylpropanenitrile (9)

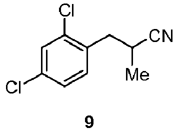

According to *General Procedure E,* to a solution of **7** (0.50 g, 0.236 mmol, 1.0 equiv) in MeOH (1.5 mL) was added magnesium turnings (0.229 g, 9.44 mmol, 40.0 equiv). The exothermic reaction which ensued after 10 min was moderated with ice bath. The reaction was stirred for 1h at 0 °C and then at room temperature overnight. The crude was purified by chromatography on silica gel (hexane / AcOEt; 95:5). Compound **9** was obtained in 96% yield (0.048 g) as transparent oil.
**¹H NMR** (400 MHz, CDCl₃) δ: 7.41 (m, 1H, Ph H-3), 7.26 (m, 1H, Ph H-6), 7.26 (m, 1H, Ph H-5), 3.03-2.93 (m, 3H, CH, CH₂), 1.39 (d, *J* = 6.9 Hz, 3H, CH₃).
**¹³C NMR** (101 MHz, CDCl₃) δ: 134.7 (Ph C-2), 134.1 (Ph C-4), 133.3 (Ph C-1), 132.2 (Ph C-6), 129.6 (Ph C-3), 127.5 (Ph C-5), 122.0 (C≡N), 37.3 (CH₂), 25.9 (CH), 17.8 (CH₃).
**HRMS (ESI),***m*/*z*:calcd. for C₁₀H₉Cl₂NNa 236.0004 [M+Na]⁺; found: 236.0007.

### 3-(2-chloro-4-methylphenyl)butanenitrile (10)

Yasuda, M., Onishi, Y., Ueba, M., Miyai, T., Baba, A. J. Org. Chem., 2001, 66, 7741-7744.

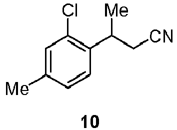

To a mixture of InCl₃ (0.016 g, 0.007 mmol, 0.2 equiv) and 3-(2-chloro-4-methylphenyl)3-hydroxy-butanenitrile (0.074 g, 0.353 mmol, 1.0 equiv) in dry DCM (1.4 mL) was added chlorodiphenylsilane (0.154 g, 0.706 mmol, 2.0 equiv) under argon. The reaction mixture was stirred for 48 h at -20 °C and then for 24 h at 0 °C. The resulting mixture was poured into Et₂O and water. The homogeneous solution was extracted with Et₂O, and the organic layer was dried over Na₂SO₄. The crude was purified by chromatography on silica gel (hexane/AcOEt, 98:2) to obtain the pure compound **10** in 40% yield (0.027g) as transparent oil.
**¹H NMR** (400 MHz, CDCl₃) δ: 7.21 (m, 1H, Ph H-3), 7.20 (m, 1H, Ph H-6), 7.09 (m, 1H, Ph H-5), 3.65 (m, 1H, CH), 2.67 (dd, *J* = 16.7, 5.5 Hz, 1H, CH₂), 2.57 (dd, 16.7, 7.4 Hz, 1H, CH₂), 2.31 (s, 3H, Ph-CH₃), 1.45 (d, *J* = 7.0 Hz, 3H, CH₃).
**¹³C NMR** (100 MHz, CDCl3) δ: 138.6 (Ph C-4),136.8 (Ph C-1), 133.1 (Ph C-2), 130.3 (Ph C-3), 128.1 (Ph C-5), 126.7 (Ph C-6), 118.2 (C=N), 31.9 (CH), 24.8 (CH₂), 20.7 (Ph-CH₃), 19.1 (CH₃).
**HRMS (ESI),** *m*/*z:*calcd. for C₁₁H₁₂ClNNa 216.0550 [M+Na]⁺; found: 216.0550.

### (2E)-3-(2,4-dichlorophenyl)-2-butenamide (E-11)

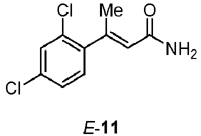

According to *General Procedure D,* a mixture of *E*-**2** (0.1 g, 0.474 mmol, 1.0 equiv) and Cu(OAc)₂ (0.086 g, 0.474 mmol, 1.0 equiv), in HOAc (5 mL) and H₂O (0.4 mL) was refluxed at 120 °C for 72 h and then poured into sat. aqueous NaCl, which was extracted with DCM. The combined organic phases were washed with H₂O, dried over Na₂SO₄, filtered and concentrated under reduced pressure. The crude was purified by chromatography on silica gel (hexane/AcOEt, 70:30). Compound *E-11* was obtained in 51% yield (0.055 g) as white solid; mp118-121 °C.
**¹H NMR** (500 MHz, CDCl₃) δ: 7.40 (s, 1H, Ph H-3), 7.23 (d, *J* = 8.2 Hz, 1H, Ph H-5), 7.11 (d, *J* = 8.0 Hz, 1H, Ph H-6), 5.77 (s, 1H, C=CH), 5.77 (s, 1H, NH₂), 5.54 (br s, 1H, NH₂), 2.44 (s, 3H, CH₃).
**¹³C NMR** (125 MHz, CDCl₃) δ: 167.9 (C=O), 151.3 (*C*C=O), 141.2 (Ph C-1), 134.0 (Ph C-4),132.2 (Ph C-2), 129.8 (Ph C-6), 129.6 (Ph C-3),127.1 (Ph C-5),122.2 (=CH-), 19.6 (CH₃).
**HRMS (ESI),** *m*/*z:*calcd. For C₁₀H₉Cl₂NNaO:251.9953 [M+Na]⁺; found:251.9956.

### 2-(2,4-dichlorophenoxy)propanenitrile (12)

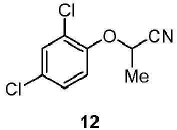

According to *General Procedure A,* starting from 2,4-dichlorophenol (0.897 g, 5.50 mmol, 1.1 equiv), 2-bromopropionitrile (0.670 g, 5.00 mmol, 1.0 equiv) and Cs₂CO₃ (2.444 g, 7.50 mmol, 1.5 equiv) in acetonitrile (30 mL), compound **12** was obtained in 84% yield (0.908 g) as white solid; mp 70-72 °C.
**¹H-NMR** (500 MHz, CDCl₃)δ: 7.43 (s, 1H, Ph H-3), 7.26 (m, 1H, Ph H-5), 7.11 (m, 1H, Ph H-6), 4.85 (q, *J* = 6.8 Hz, 1H, CH), 1.85 (d, *J* = 6.8 Hz, 3H, CH₃). **¹³C-NMR** (125 MHz, CDCl₃)δ: 150.8 (Ph C-1), 130.6 (Ph C-3), 129.6 (Ph C-4), 128.1 (Ph C-5), 126.0 (Ph C-2), 118.9 (Ph C-6), 117.6 (-C=N), 64.8 (CH), 19.8 (CH₃). **HRMS (APCI),***m*/*z:*calcd. for C₉H₇Cl₂NOH⁺ 215.9977 [M+H]⁺; found 215.9977.

### (2Z)-3-(2,4-dichlorophenyl)-2-butenamide (Z-11)

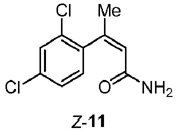

According to *General Procedure D,* a mixture of *Z***-2** (0.052 g, 0.246 mmol, 1.0 equiv) and Cu(OAc)₂ (0.045 g, 0.246 mmol, 1.0 equiv), in HOAc (2.5 mL) and H₂O (0.4 mL) was refluxed at 120 °C for 72 h and then poured into saturated aqueous NaCl, which was extracted with DCM. The combined organic phases were washed with H₂O, dried over Na₂SO₄, filtered and concentrated under reduced pressure. The crude was purified by chromatography on silica gel (hexane/AcOEt, 70:30). Compound *Z*-**11** was obtained in 42% yield (0.024 g) as white solid; mp 137-140 °C.
**¹H NMR** (400 MHz, CDCl₃) δ: 7.43 (d, *J* = 2.1 Hz, 1H, Ph H-3), 7.26 (dd, *J* = 8.2, 2.1 Hz, 1H, Ph H-5), 7.09 (d, *J* = 8.2 Hz, 1H, Ph H-6), 6.00 (q, *J* = 1.5 Hz, 1H, C=CH), 5.44 (br s, 1H, NH₂), 5.15 (br s, 1H, NH₂), 2.10 (d, *J* = 1.5 Hz, 3H, CH₃).
**¹³C NMR** (101 MHz, CDCl₃) δ: 167.0 (C=O), 146.9 (*C*C=O), 138.1 (Ph C-1), 134.1 (Ph C-4), 131.8 (Ph C-2), 129.6 (Ph C-3), 129.4 (Ph C-6), 127.5 (Ph C-5), 123.2 (CH), 25.6 (CH₃).
**HRMS (ESI),** *m*/*z:* calcd. For C₁₀H₉Cl₂NNaO: 251.9953 [M+Na]⁺; found: 251.9958.

### 2-(2-chloro-4-methylphenoxy)propanenitrile (13)

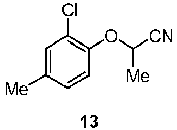

According to *General Procedure A,* starting from 2-chloro-4-methylphenol (0.784 g, 5.50 mmol, 1.1 equiv), 2-bromopropionitrile (0.670 g, 5.00 mmol, 1.0 equiv) and Cs₂CO₃ (2.444 g, 7.50 mmol, 1.5 equiv) in acetonitrile (30 mL), compound 13 was obtained in 88% yield (0.864 g) as brownish solid; mp 36-38 °C.
**¹H-NMR** (500 MHz, CDCl₃) δ: 7.23 (s, 1H, Ph H-3), 7.07 (s, 1H, Ph H-6), 7.06 (s, 1H, Ph H-5), 4.84 (q, *J* = 6.8 Hz, 1H, CH), 2.31 (s, 3H, Ph-CH₃), 1.83 (d, *J* = 6.8 Hz, 3H, CH₃).
**¹³C-NMR** (125 MHz, CDCl₃) δ: 149.9 (Ph C-1), 135.1 (PhC-4), 131.2 (Ph C-3),128.5 (Ph C-5), 124.8 (Ph C-2), 118.7 (Ph C-6), 118.1 (-C=N), 65.0 (CH), 20.5 (CH₃), 19.9 (Ph-CH₃).
**HRMS (APCI),** *m*/*z:*calcd. for C₁₀H₁₀ClNOH⁺ 196.0524 [M+H]⁺; found 196.0526.

### (R)-(+)-2-(2,4-dichlorophenoxy)propanenitrile ((R)-12)

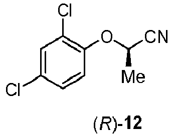

To a solution of dry DMF in dry THF at room temperature oxalyl chloride (0.049 g, 0.386 mmol, 2.0 equiv) was added dropwise. After 5 min a solution of (R)-2-(2,4-dichlorophenoxy)propanamide (0.045 g, 0.193 mmol, 1.0 equiv) in THF was added and the reaction mixture was stirred for 45 min at room temperature. EtOAc was poured and the mixture was filtered and concentrated under reduced pressure. Compound (*R***)-12** was obtained in 95% yield (0.039 g) as a white solid; mp 70-72 °C.
**¹H-NMR** (500 MHz, CDCl₃) δ: 7.43 (s, 1H, Ph H-3), 7.26 (m, 1H, Ph H-5), 7.11 (m, 1H, Ph H-6), 4.85 (q, *J* = 6.8 Hz, 1H, CH), 1.85 (d, *J* = 6.8 Hz, 3H, CH₃).
**¹³C-NMR** (125 MHz, CDCl₃) δ: 150.8 (Ph C-1), 130.6 (Ph C-3), 129.6 (Ph C-4), 128.1 (Ph C-5), 126.0 (Ph C-2), 118.9 (Ph C-6), 117.6 (-C≡N), 64.8 (CH), 19.8 (CH₃) **HRMS (APCI),** *m*/*z:*calcd. for C₉H₇Cl₂NOH⁺ 215.9977 [M+H]⁺; found: 215.9977 [α]_{D}+114 (c 0.5, CHCl₃).
**HPLC analysis:** Chiralpak IA Column, λ 250 nm, eluent: *n*-hexane / *i*-propanol 95/5. Flow:1 mL/min.

### 2-(2,4-dimethylphenoxy)propanenitrile (14)

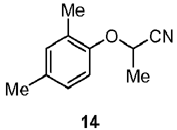

According to *General Procedure A,* starting from 2,4-dimethylphenol (0.672 g, 5.50 mmol, 1.1 equiv), 2-bromopropionitrile (0.670 g, 5.00 mmol, 1.0 equiv) and Cs₂CO₃ (2.444 g, 7.50 mmol, 1.5 equiv) in acetonitrile (30 mL), compound **14** was obtained in 94% yield (0.824 g) as a brown viscous liquid.
**¹H-NMR** (500 MHz, CDCl₃)δ: 7.01 (s, 1H, Ph H-3), 6.99 (m, 1H, Ph H-5), 6.89 (m, 1H, Ph H-6), 4.81 (q, *J* = 6.8 Hz, 1H, CH), 2.29 (s, 3H, Ph C4-CH₃), 2.23 (s, 3H, Ph C2-CH₃), 1.80 (d, *J* = 6.8 Hz, 3H, CH₃).
**¹³C-NMR:** (125 MHz, CDCl₃)δ: 152.5 (Ph C-1), 132.4 (Ph C-4), 132.1 (Ph C-3), 127.7 (Ph C-2), 127.2 (Ph C-5), 118.6 (-C≡N), 113.7 (Ph C-6), 63.1 (CH), 20.5 (Ph C4-CH₃), 20.0 (CH₃), 16.0 (Ph C2-CH₃).
**HRMS (APCI):** *m*/*z:*calcd. for C₁₁H₁₃NOH⁺ 176.1070 [M+H]⁺; found 176.1073.

### (R)-(+)-2-(4-chloro-2-methylphenoxy)propanenitrile ((R)-15)

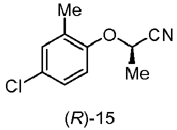

In a flask equipped with water condenser, Et₃Al 2M (4.65 mL, 9.3 mmol, 7.5 equiv) was added to a solution of NH₃ (0.5 M in dioxane, 19.84 mL, 9.92 mmol, 8.0 equiv) in toluene (10.0 mL) at 0 °C and the reaction mixture was stirred for 1h at the same temperature. (*R*)-(+)-ethyl-2-(4-chloro-2-methylphenoxy)propanoate (0.300g, 1.24 mmol, 1.0 equiv) was then added and the mixture was refluxed for 3h. H₂O was added and the organic phase extracted with AcOEt. The organic layer was washed with saturated solution of NaCl, dried over Na₂SO₄, filtered and concentrated under reduced pressure. The crude was purified by chromatography on silica gel (hexane/AcOEt; 95:5). Compound (*R*)-**15** was obtained in 56% yield (0.039 g) as a light brown solid; mp 45-48 °C.
**¹H-NMR** (500 MHz, CDCl₃) δ: 7.17 (s, 1H, Ph H-3), 7.15 (m, 1H, Ph H-5), 6.89 (m, 1H, Ph H-6), 4.82 (q, *J* = 6.8 Hz, 1H, CH), 2.22 (s, 3H, Ph-CH₃), 1.81 (d, *J* = 6.8 Hz, 3H, CH₃).
**¹³C-NMR** (125 MHz, CDCl₃) δ: 153.1 (Ph C-1), 131.2 (Ph C-3), 129.9 (Ph C-2),127.8 (Ph C-4), 126.7 (Ph C-5), 118.1 (-C≡N), 114.5 (Ph C-6), 63.0 (CH), 20.0 (CH₃), 16.0 (Ph-CH₃).
**HRMS (APCI),***m*/*z:*calcd. for C₁₀H₁₀ClNOH⁺: 196.0524 [M+H]⁺; found: 196.0526. **[α]_{D}**+73 (c 0.5, CHCl₃).

### 5-(1-(3,5-dimethylphenyl)prop-1-en-2-yl)-1H tetrazole (16)

According to *General Procedure F,* diethylaluminiumazide (Et₂AlN₃) was prepared *in situ* from sodium azide (0.423 g, 6.50 mmol, 4.3 equiv) and diethylaluminium chloride (7.22 mL, 6.50 mmol, 4.3 equiv, 0.9 M in toluene) in xylene (9 mL). Nitrile **18** (0.257 g, 1.50 mmol, 1.0 equiv) was added to the suspension of Et₂AlN₃ and the resulting mixture was stirred for 48 h at 140 °C. Following the work-up procedure reported above sodium nitrite (1.35 g, 19.50 mmol, 13.0 equiv) in sodium hydroxide (15% (w/w), 5.18 mL, 19.50 mmol, 13.0 equiv) were used. Compound **16** was obtained in 40% yield (0.130 g) as a brownish solid; mp 116 °C with decomposition; *E:Z* ratio = 71:29.

### E-16:

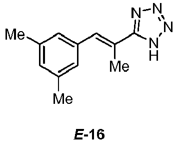

**¹H-NMR** (500 MHz, CDCl₃) δ: 7.68 (s, 1H, =CH-),7.04 (s, 2H, Ph H-2,6),6.97 (s, 1H, Ph H-4), 2.46 (s, 3H, CH₃), 2.33 (s, 6H, Ph-CH₃).
**¹³C-NMR** (125 MHz, CDCl₃) δ:158.5 (tetrazole-C), 138.0 (Ph C-3,5), 135.5 (=CH-), 135.2 (Ph C-1), 130.1 (Ph C-4), 127.3 (Ph C-2,6), 120.2(=C-), 21.3 (Ph-CH₃), 15.8 (CH₃).

### Z-16:

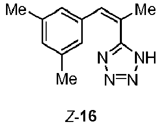

**¹H-NMR** (500 MHz, CDCl₃) δ: 7.06 (s, 1H, =CH-), 7.01 (s, 1H, Ph H-4), 6.76 (s, 2H, Ph H-2,6), 2.42 (s, 3H, CH₃), 2.28 (s, 6H, Ph-CH₃).
**¹³C-NMR** (125 MHz, CDCl₃) δ: 153.8 (tetrazole-C), 139.6 (Ph C-3,5), 136.6 (=CH-), 135.8 (Ph C-1), 130.6 (Ph C-4), 125.2 (Ph C-2,6), 121.5 (=C-), 21.8 (CH₃), 21.2 (Ph-CH₃).
**HRMS (APCI),***m*/*z:*calcd. for C₁₂H₁₃N₄NaH⁺: 237.1111 [M+H]⁺; found: 237.1110.

### 2-(4-chloro-2-methylphenoxy)propanenitrile (15)

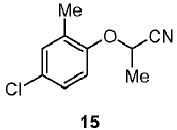

According to *General Procedure A,* starting from 4-chloro-2-methylphenol (0.784 g, 5.50 mmol, 1.1 equiv), 2-bromopropionitrile (0.670 g, 5.00 mmol, 1.0 equiv) and Cs₂CO₃(2.444 g, 7.50 mmol, 1.5 equiv) in acetonitrile (30 mL), compound **15** was obtained in 86% yield (0.844 g) as light brown solid; mp45-48 °C.
**¹H-NMR** (500 MHz, CDCl₃)δ: 7.17 (s, 1H, Ph H-3), 7.15 (m, 1H, Ph H-5), 6.89 (m, 1H, Ph H-6), 4.82 (q, *J*= 6.8 Hz, 1H, CH), 2.22 (s, 3H, Ph-CH₃), 1.81 (d, *J* = 6.8 Hz, 3H, CH₃).
**¹³C-NMR** (125 MHz, CDCl₃)δ: 153.1 (Ph C-1), 131.2 (Ph C-3), 129.9 (Ph C-2), 127.8 (Ph C-4), 126.7 (Ph C-5), 118.1 (-C≡N), 114.5 (Ph C-6), 63.0 (CH), 20.0 (CH₃), 16.0 (Ph-CH₃).
**HRMS (APCI),***m*/*z:*calcd. for C₁₀H₁₀ClNOH⁺: 196.0524 [M+H]⁺; found: 196.0526.

### 5-(1-(2,4-dichlorophenoxy)ethyl)-1H-tetrazole (17)

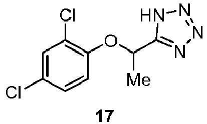

According to *General Procedure F,* diethylaluminiumazide was prepared *in situ* from sodium azide (0.845 g, 13.0 mmol, 4.3 equiv) and diethylaluminium chloride (14.44 mL, 13.0 mmol, 4.3 equiv, 0.9 M in toluene) in xylene (18 mL). Nitrile **12** (0.645 g, 3.0 mmol, 1.0 equiv) was added to the suspension of Et₂AlN₃ and tetrazole **17** was obtained after stirring the mixture for 20 h at 60 °C. For work-up was used sodium nitrite (2.69 g, 39.0 mmol, 13.0 equiv) in sodium hydroxide (15 % (w/w), 10.37 mL, 39.0 mmol, 13.0 equiv). Compound **17** was obtained in 76% yield (0.591 g) as white solid; mp115 °C.
**¹H-NMR** (400 MHz, CDCl₃)δ: 7.38 (d, *J* = 2.5 Hz, 1H, Ph H-3), 7.16 (dd, *J* = 8.8, 2.5 Hz, 1H, Ph H-5), 6.92 (d, *J* = 8.8 Hz, 1H, Ph H-6), 5.79 (q, *J* = 6.6 Hz, 1H, CH), 1.85 (d, *J* = 6.6 Hz, 3H, CH₃).
**¹³C-NMR** (100 MHz, CDCl₃)δ: 157.7 (tetrazole-C), 150.8 (Ph C-1), 130.5 (Ph C-3), 128.5 (Ph C-4), 128.1 (Ph C-5), 125.2 (Ph C-2), 117.7 (Ph C-6), 70.4 (CH), 20.4 (CH₃) **HRMS (ESI),** *m*/*z:*calcd. forC₉H₈Cl₂N₄NaO 280.9994 [M+Na]⁺; found: 280.9996.

### 3-(3,5-dimethylphenyl)-2-methylacrylonitrile (18)

According to *General Procedure B,* a mixture of 3,5-dimethylphenylboronic acid (0.75 g, 5.00 mmol, 1.2 equiv), *cis*/*trans*3-bromo-2-methylacrylonirile (0.608 mg, 4.17 mmol, 1.0 equiv), Cs₂CO₃ (4.073 g, 12.50 mmol, 3.0 equiv) and bis(triphenylphosphine)palladium(II)dichloride (0.146 g, 0.21 mmol, 5 mol%,) in acetonitrile (50 mL) was refluxed for 48 h. The residue was purified by chromatography on silica gel (petrol ether/AcOEt, 95:5). Compound **18** was obtained in 77% yield (0.548 g) as a yellowish viscous liquid; E:Z ratio =52:48.

### E-18:

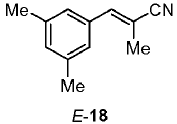

**¹H-NMR** (500 MHz, CDCl₃) δ: 7.15 (s, 1H, =CH-), 7.01 (s, 1H, Ph H-4), 6.94 (s, 2H, Ph H-2,6), 2.34 (s, 6H, Ph-CH₃), 2.14 (s, 3H, CH₃).
**¹³C-NMR** (125 MHz, CDCl₃)δ: 144.3 (=CH-),138.2 (Ph C-3,5),134.0 (Ph C-1),131.0 (Ph C-4), 127.1 (Ph C-2,6), 121.5 (-C≡N), 109.1 (C-C≡N), 21.2 (Ph-CH₃), 16.8 (CH₃).

### Z-18:

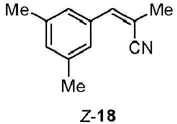

**¹H-NMR** (500 MHz, CDCl₃)δ: 7.32 (s, 2H, Ph H-2,6), 7.02 (s, 1H, Ph H-4), 6.87 (s, 1H, =CH-), 2.34 (s, 6H, Ph-CH₃), 2.14 (s, 3H, CH₃).
**¹³C-NMR** (125 MHz, CDCl₃)δ: 144.8 (=CH-),138.3 (Ph C-3,5),133.7 (Ph C-1),131.5 (Ph C-4), 126.2 (Ph C-2,6), 119.3 (-C≡N), 105.4 (C-C≡N), 22.2 (CH₃), 21.2 (Ph-CH₃). **HRMS (APCI),***m*/*z:*calcd. for C₁₂H₁₂NNaH⁺: 194.0940 [M+H]⁺; found: 194.0943.

### 2) Biochemistry and molecular biology

### Materials and Methods

### Expression of GABA_{A} receptors in Xenopus laevis oocytes and two-microelectrode voltage-clamp experiments

Preparation of stage V-VI oocytes from *Xenopus laevis* and synthesis of capped runoff poly(A) cRNA transcripts from linearized cDNA templates (pCMV vector) was performed as described in Khom *et. al,* Mol. Pharmacol. 2006.. Female *Xenopus laevis* frogs were anesthetized by 15 min incubation in a 0.2% MS-222 (methane sulfonate salt of 3-aminobenzoic acid ethyl ester; Sigma Aldrich, Vienna, Austria) solution before removal of parts of the ovaries. Follicle membranes from isolated oocytes were enzymatically digested with 2mg/mL collagenase (Type 1A, Sigma-Aldrich, Vienna, Austria). Mutations β_{1S265N}, β_{3N265S} and β_{3F289S} were introduced by site-directed mutagenesis using the QuikChange mutagenesis kit (Agilent Technologies, Vienna, Austria). The coding regions of plasmids were sequenced before experimental use. After cDNA linearization, capped cRNA transcripts were produced using the mMESSAGE mMACHINE® T7 transcription kit (Life Technologies). Selected oocytes were injected with 10-50nL of DEPC-treated water (diethyl pyrocarbonate, Sigma, Vienna, Austria) containing the different GABA_{A} cRNAs at a concentration of approximately 200-3000pg/nL/subunit. To ensure expression of the γ_{2S} subunit in the case of α₁β_{2/3}γ_{2S}, α₁β_{1S265N}γ_{2S}, α₁β_{3F289S}γ_{2S} receptors, cRNAs were mixed in a ratio of 1:1:10. For expression of receptors composed of α₁β_{3N265S}γ_{2S} and α₁β₁γ_{2S} cRNAS were mixed in a ratio of 3:1:10. The amount of cRNAs was determined by means of a NanoDrop ND-1000 (Kisker-Biotech, Steinfurt, Germany). Oocytes were stored at +18°C in ND96 all from Sigma-Aldrich, Vienna, Austria).

Chloride currents through GABA_{A} receptors (I_{GABA}) were measured at room temperature (+21±1°C) by means of the two-microelectrode voltage clamp technique making use of a TURBO TEC-05X amplifier (npi electronic, Tamm, Germany). I_{GABA} were elicited at a holding potential of -70mV. Data acquisition was carried out by means of an Axon Digidata 1322A interface using pCLAMP v.10 (Molecular Devices, Sunnyvale, CA, USA). ND96 solution was used as bath solution. Microelectrodes were filled with 3M KCl and had resistances between 1 and 3MΩ.

### Fast Perfusion System

GABA and drugs were applied by means of a fast perfusion system as described in the prior art; drug or control solutions were applied by means of a TECAN Miniprep 60 enabling automation of the experiments (ScreeningTool, npi electronic, Tamm Germany). To elicit I_{GABA}, the chamber was perfused with 120µL of GABA- or compound-containing solutions, respectively, at a volume rate of 300µL/s. To account for possible slow recovery from increasing levels of desensitization in the presence of high compound concentrations, the duration of washout periods was extended stepwise, i.e. 1min (GABA EC₃₋₇) to 1.5min (co-application of GABA EC₃₋₇ in the presence ≤1µM compound;) to 2.5min (co-application of GABA EC₃₋₇ in the presence of ≤10µM compound) to 5min (co-application of GABA EC₃₋₇ and ≤100µM compound) to 15min (GABA EC₃₋₇ in the presence of 300-500µM compound). Oocytes with maximal current amplitudes >5µA were discarded to exclude voltage-clamp errors.

### GABA_{A} receptors

Enhancement of chloride currents by modulators of the GABA_{A} receptor was measured at a GABA concentration eliciting between 3% and 7% of the maximal current amplitude (EC₃₋₇). The GABA EC₃₋₇ was determined for each oocyte individually. Enhancement of the chloride current was defined as (I_{(GABA+compound)}/I_{GABA}) - 1, where I_{(GABA+compound)} is the current response in the presence of a given compound and I_{GABA} is the control GABA current. I_{GABA-max} reflects the maximal I_{GABA} enhancement. Concentration-response curves were generated and the data were fitted by nonlinear regression analysis using Origin Software (OriginLab Corporation, USA). Data were fitted to the following equation: y= min + (max-min) * x^{nH} / (k^{nH}+x^{nH}).
In this equation, k corresponds to the EC₅₀ value, x-values are logs of concentration, and n_{H} stands for the Hill coefficient. Each data point represents the mean ± SEM from at least 3 oocytes and ≥2 oocyte batches.
Statistical significance was calculated using student's t-test or one-way ANOVA followed by a post-hoc mean comparison with Bonferroni (OriginLab Corporation, USA or GraphPad, La Jolla, USA). P- values of <0.05 were accepted as statistically significant. All data are given as mean ± SEM.

### Electrophysiology on neuronal cells

All recordings were done at room temperature (20-24°C). Patch pipettes were made with a Sutter P97 horizontal puller (Sutter Instruments, Novato, CA, U.S.A.) using borosilicate glass capillaries (GB150-8P; Science Products, Hofheim, Germany). Tip resistances were between 2 and 5 MΩ. Recordings were performed using the whole-cell patch clamp method. The internal solution contained (mM): KCl (140), CaCl₂ (2), MagCl₂ (0.7), EGTA (10) and HEPES (10) (pH adjusted to 7.3 with KOH). The external solution consisted of (mM): NaCl (140), glucose (10), CaCl₂ (2.5), MgCl₂ (2) and HEPES (10) (pH adjusted to 7.3 with 3mM KOH).

For voltage clamp recordings, an Axopatch 200B amplifier was used (Klinger F, Geier P, Dorostkar MM, Chandaka GK, Yousuf A, Salzer I, Kubista H, Boehm S (2012),Br J Pharmacol 166:1631-1642). For the determination of tonic currents, the internal solution was composed of (mM): 135 CsCl, 10 HEPES, 10 EGTA, and 1 MgCl₂ (adjusted to pH 7.3 with CsOH). For GABA-evoked currents, the internal solution contained (mM): 140 KCl, 2 CaCl₂, 0.7 MgCl₂, 10 EGTA, and 10 HEPES (pH was adjusted to 7.3 with KOH). For the recording of tonic currents, 6-cyano-7-nitroquinoxaline-2,3-dione (CNQX, 10 µM) and tetrodotoxin (1 µM) were added to suppress glutamatergic neurotransmission and action potential propagation, respectively.

### In vivo characterization of seizure threshold

Only male C57BL/6N mice (age 3-6 months) were used for determination of the seizure threshold. Intraperitoneal injection of control or compound-containing solutions was done 30 min before the test. Indicated doses in the results represent milligrams per kilogram body weight. Seizure threshold was determined by pentylenetetrazole (PTZ) tail-vein infusion on freely moving animals at a rate of 100 µl/min (10 mg/ml PTZ in saline, pH = 7.4). Infusion was stopped when animals displayed generalized clonic seizures. Animals were immediately killed by cervical displacement after onset of seizures. The seizure threshold dose was calculated from the infused dose in relation to body weight (mg/kg).

### Results

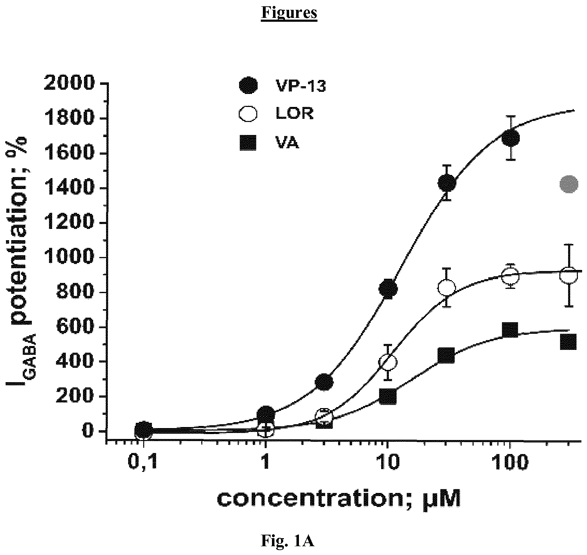

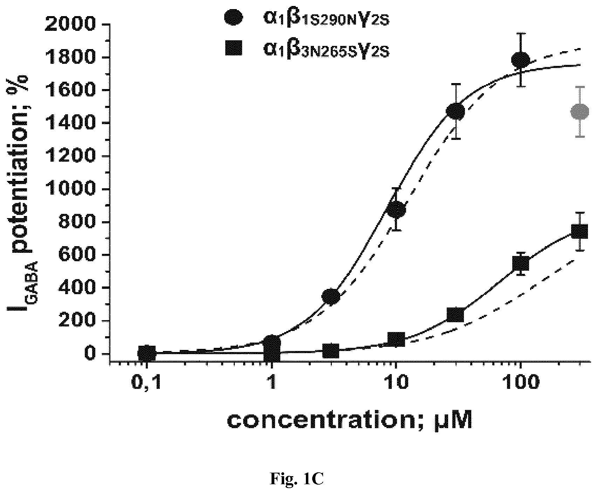

Functional effects of compound Z-2 (VP-13) were investigated on recombinant GABA_{A} receptors expressed in *Xenopus laevis* oocytes. Modulation of I_{GABA} by compound Z-2 (VP-13) was first studied on GABA_{A} channels composed of α₁β₃γ_{2S} subunits (Eₘₐₓ = 1896.4 ± 114.3 %; EC₅₀ = 12.8 ± 1.7 µM; n = 7). As depicted in Fig. 1A compound *Z***-2** (VP-13) exhibited significantly higher efficacy compared to prior art agent LOR (Eₘₐₓ = 931.6 ± 28.0; n = 5; p < 0.001) and VA (Eₘₐₓ = 603.8 ± 42.7 %; n = 7; p < 0.001). Additionally, compound *Z-***2** (VP-13) showed significantly higher potency than VA (EC₅₀: 19.3 ± 2.0 µM; n = 7; p < 0.05).
A strong dependence of the modulatory effect of compound *Z-***2** (VP-13) on the β subunit composition of the receptor was observed. Fig. 1B illustrates the effect of compound *Z-***2** (VP-13) on I_{GABA} through GABA_{A} channels with different β subunit isoforms. While the activity on β_{2/3} containing channels (α₁β₃γ_{2S:} Eₘₐₓ = 1896.4 ± 114.3 %; EC₅₀ = 12.8 ± 1.7; n = 7; α₁β₃γ_{2S:} Eₘₐₓ = 1804.9 ± 153.0; EC₅₀ = 9.9 ± 2.5 µM; n = 3) was comparable replacing β_{2/3} by β₁ drastically reduced the sensitivity of the resulting GABA_{A} channels (Eₘₐₓ and EC₅₀ not determined). The point mutation β_{3N265S} drastically reduced the stimulatory effect of compound *Z-***2** (VP-13). It is to be noted that the corresponding mutation in β1 (β_{1S290N}) however induced sensitivity for compound *Z-***2** (VP-13) (Fig. 1C). These findings suggest an involvement of this residue in either binding of compound *Z-***2** (VP-13) or transduction of the effect.

Thus, it can be gathered from Fig. 1A, that the compound according to the invention shows a stronger modulation of the GABA_{A} receptor response, and specifically a higher potentiation of the I_{GABA}, than the agents known in the prior art, and thus a higher efficacy. The compound is also more potent than, for example, VA, as indicated by a lower EC₅₀ value, i.e. the concentration of a drug which induces a response halfway between the baseline and maximum after a specified exposure time. Furthermore, Fig. 1B shows that the compound according to the present invention may be especially effective in modulating the response of selected GABA_{A} receptors, namely those GABA_{A} receptors comprising β₂ and/or β₃ subunit(s). This may allow for targeting specific brain circuits. Furthermore, β₂ and/or β₃ subunit selectivity may allow for a medical treatment with less negative side-effects for a patient.

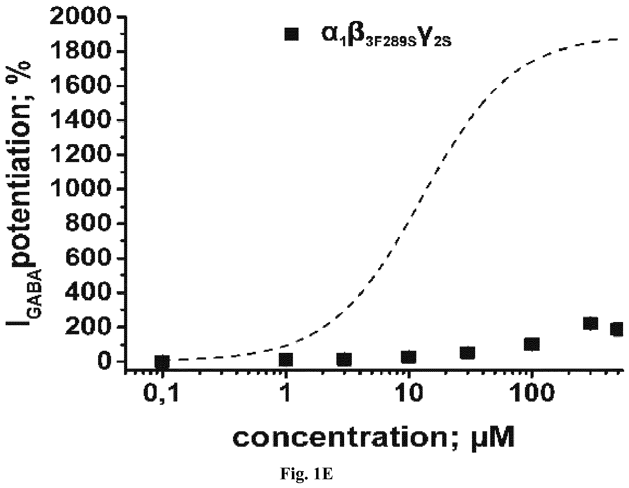

The point mutation studies depicted in Fig. 1C may give insight into the binding mechanism of the compound according to the invention. For example, the replacement of asparagine in position 265 of protein subunit β3 is accompanied by a reduction of the stimulatory effect of compound *Z***-2**. Thus, it appears that the carbamoyl group of the asparagine residue might be responsible for an interaction with compound *Z***-2** e.g. by a hydrogen-bond donor/acceptor interaction. Furthermore, Fig. 1E) shows that the replacement of phenylalanine F289 of subunit β₃ results in a reduction of the I_{GABA} modulatory effect of compound *Z***-2** (VP-13). It appears that the activity of compound 2 relies inter alia on the interaction of the disubstituted phenyl group interacts with the benzene core of phenylalanine F289 of subunit β3 via π-stacking, i.e. an interaction of the π- electron system of the aromatic rings. The subunit β3 and subunit β₂ are widely conserved, i.e. they show very high similarities in their amino acid code. Thus, similar conclusions may likely be made for an interaction of compound *Z***-2** with a subunit β₂ of the GABA_{A} receptor.

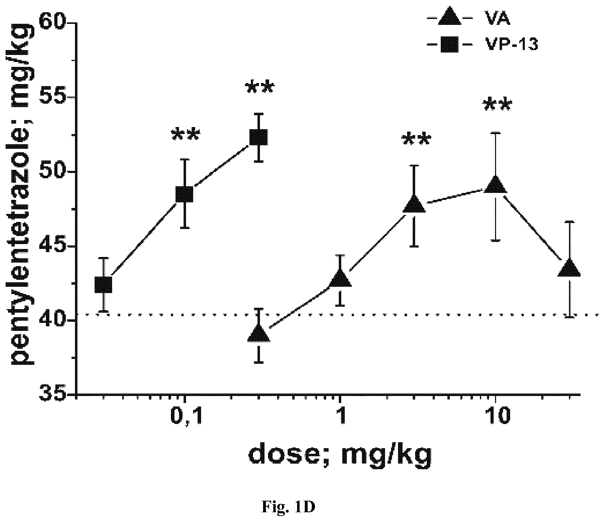

In order to get insights into the anticonvulsant properties of compound *Z-***2** (VP-13), the effect of compound *Z-***2** on changes in seizure threshold upon pentylenetetrazole (PTZ) tail vein infusion was studied. The dose-dependent effect of compound *Z***-2** (VP-13) on seizure threshold was determined. As depicted in Fig. 1D, pronounced seizure threshold elevation was observed after application of compound *Z***-2** (VP-13) at a dose of 0.1 or 0.3 mg/kg body weight (control: 39.5 ± 0.8 mg/kg PTZ, n = 6 vs. VP-13 0.1 mg/kg: 48.5 ± 2.3 mg/kg PTZ, n = 4, p < 0.01 and VP-13 0.3 mg/kg: 52.3±1.6 mg/kg PTZ, n = 4; p < 0.001, respectively). Thus, compound *Z***-2** (VP-13) elevated the PTZ-induced seizure threshold more potently compared to prior art agent VA. An elevation of the seizure threshold by a drug is usually taken as an indicator for its anticonvulsant properties. The compounds according to the invention are more effective in elevating the seizure threshold than prior art agents, and have thus better anticonvulsant properties.

### Effects of VP-13 on cultured neuronal cells

The effects of compound *Z*-**2** (VP-13) on native GABA_{A} receptors were characterised on primary cultures of dissociated hippocampal neurons. These neuronal cells express a wide variety of different GABA_{A} receptor subunits (Sieghart W, Sperk G (2002), Curr Top Med Chem 2:795-816) and phasic and tonic GABAergic inhibition mediated by synaptic and extrasynaptic receptors can be characterised (Yeung JYT, Canning KJ, Zhu G, Pennefather P, MacDonald JF, Orser BA (2003), Mol Pharmacol 63:2-8).

As illustrated in Fig. 2B and C, compound *Z***-2** (VP-13) delays the decay of mIPSCS (normalized: 213.8 ± 41.0%; n=5; p<0.05) compared to control while there was no effect observed on amplitude or frequency. A prolonged decay of mIPSCS indicates a synaptic effect of compound *Z-***2,** and especially a postsynaptic effect. The synaptic effect is most likely due to an interaction with GABA_{A} subtypes comprising β₃ subunit(s), which are highly abundant in the synaptic cleft of neurons. The prolonged opening of the GABA-controlled ion channel results in a stronger neuronal transmission. This is another indicator for the activation of a synaptic GABA_{A} receptor, which mediates phasic currents.

Furthermore, in the presence of compound *Z-***2** (VP-13) holding currents were shifted towards more negative values (Fig 2A, D). It is hypothesized that this shift is due to activation of extrasynaptic GABA_{A} receptors, i.e. receptors not directly located in the synaptic cleft, which mediate tonic inhibition of neuronal excitability.

### Further compounds

Table 6 shows the I_{GABA} pot. [%] for tested compounds according to formula (I) for a GABA_{A} receptor comprising α1β3γ3s subunits at a compound concentration of 30 µM and 100 µM as well as the EC₅₀ and E_{Max} values for the compounds.

**Table 6:.**

| **Structure** | **Compound No.** | **I_{GABA} pot; % α1β3γ2s (30µM)** | **I_{GABA} pot; % α1β3γ2s (100µM)** | **EC₅₀** | **Eₘₐₓ** |
|---|---|---|---|---|---|
| 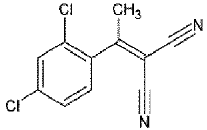 | **1** | 1488 ± 140 | 1474 ± 153 | 12.5 ± 4.3 | 1931 ± 324 |
| 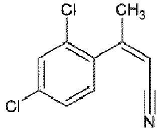 | *Z***-2 (VP-13)** | 1040 ± 122 | 1223 ± 140 | 12.8 ± 1.7 | 1896 ± 114 |
| 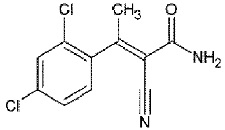 | **3(*E*/*Z* mixture)** | 740 ± 37 | 1911 ± 186 | 67 ± 9 | 3114 ± 242 |
| 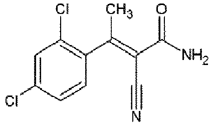 | *E***-3** | 589 ± 52 | 2181 ± 93 | 55 ± 13 | 2685 ± 347 |
| 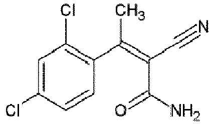 | *Z***-3** | 592 ± 57 | 1085 ± 102 | 21 ± 8 | 999 ± 200 |
| 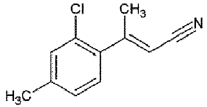 | **4 (*E*/*Z* mixture)** | 463 ± 23 | 1103 ± 56 | 41.5 ± 10.5 | 1626 ± 184 |
| 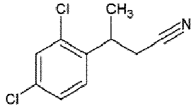 | **5** | 383 ± 48 | 1055 ± 98 | | |
| 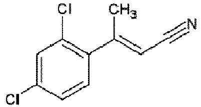 | *E*-**2** | 278 ± 84 | 1110 ± 119 | 100 ± 35 | 1946 ± 352 |
| 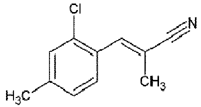 | *E*-**6** | 260 ± 43 | 1155 ± 124 | 44 ± 10.3 | 1268 ± 149 |
| 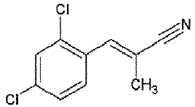 | *E***-7** | 258 ± 68 | 1130 ± 102 | 91 ± 36 | 1837 ± 363 |
| 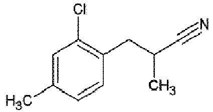 | **8** | 262 ± 22 | 931 ± 54 | 65.7 ± 27.8 | 1332 ± 255 |
| 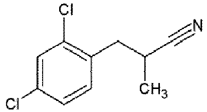 | **9** | 220 ± 34 | 840 ± 69 | | |
| 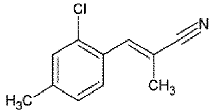 | **6 (*E*/*Z* mixture)** | 221 ± 34 | 788 ± 67 | 65.8 ± 5.7 | 1635 ± 99.6 |
| 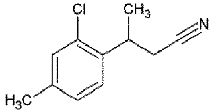 | **10** | 188 ± 22 | 545 ± 57 | | |
| 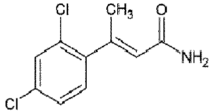 | *E-***11** | 175 ± 15 | 603 ± 39 | | |
| 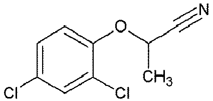 | **12** | 125 ± 16 | 460 ± 43 | 63.8 ± 26.1 | 737.5 ± 111.7 |
| 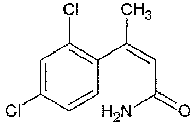 | *Z*-**11** | 110 ± 6 | 397 ± 10 | | |
| 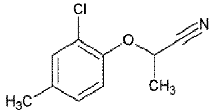 | **13** | 110 ± 12 | 516 ± 58 | 143.9 ± 27.5 | 1087 ± 101 |
| 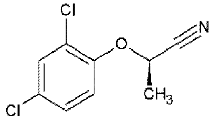 | (*R*)-**12** | 91 ± 8 | 377 ± 23 | | |
| 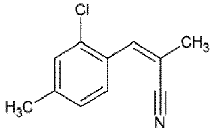 | *Z***-6** | 36 ± 7 | 256 ± 45 | | |
| 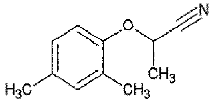 | **14** | 51 ± 3 | 238 ± 35 | | |
| 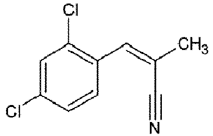 | *Z***-7** | 34 ± 24 | 199 ± 92 | | |
| 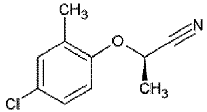 | (*R*)-**15** | 61 ± 8 | 204 ± 29 | | |
| 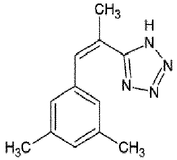 | **16 (*E*/*Z* mixture)** | 48 ± 3 | 138 ± 6 | | |
| 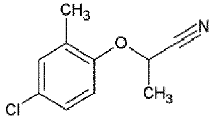 | **15** | 44 ± 11 | 175 ± 27 | | |
| 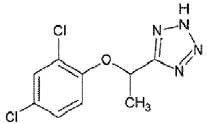 | **17** | 36 ± 8 | 84 ± 22 | | |
| 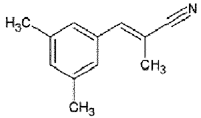 | *E***-18** | 41 ± 7 | 82 ± 21 | | |

All exemplified compounds containing an olefinic double bond refer to the pure double bond isomer as drawn in the column "structure", unless indicated otherwise. A "pure double bond isomer" in the meaning of the present invention refers to a compound having a ratio of the corresponding double bond isomers of >95:5. The indication "(*E*/*Z* mixture)" in the column "Compound No." means that the compound was used with a ratio of its double bond isomers being <95:5. The corresponding double bond isomers are assigned to the E or Z isomer according to the IUPAC principles. All exemplified compounds containing a stereogenic center were used in racemic form, unless indicated otherwise in the drawing and by a prefix to the number of the compound. In case an enantiomerically enriched compound was used, the enantiomeric enrichment (ee) was >95%.

Table 6 shows the structure-activity relationship and supports the above-mentioned trends for the specifically preferred embodiments. For example, direct comparison of compound *Z***-2** and compound *E***-2** shows that a switch from the *E-* to the *Z*-isomer, i.e. a switch from CN as substituent R⁴ to R³ leads to a better activity of the compound.

It can be gathered from Fig. 3A to 3F, that compounds **1, 3** and **4** also show a selectivity for GABA_{A} receptors comprising β₂ and/or β₃ subunit(s).

## Claims

1. A pharmaceutical composition for use as a medicament, comprising a pharmaceutically effective amount of a compound of the general formula (I) 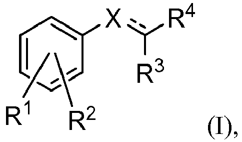 wherein
R¹ to R⁴ are independently selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups,
wherein R⁶ to R¹³ are independently selected from the group consisting of H, substituted or unsubstituted C₁ to C₆ alkyl and substituted or unsubstituted C₆ to C₁₄ aryl groups;
X is C-R⁵ or O, wherein R⁵ is selected from the group consisting of H, substituted or unsubstituted C₁ to C₆ alkyl groups and substituted or unsubstituted C₆ to C₁₄ aryl groups, wherein the dashed line represents an optional double bond, if X is C-R⁵;
or a salt of said compound according to formula (I).

2. The pharmaceutical composition for the use according to claim 1, wherein the compound is selected from the general formula (II) 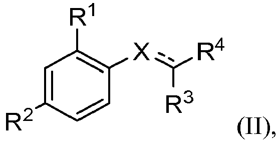 wherein
R¹ to R⁴ are independently selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups,
wherein R⁶ to R¹³ are independently selected from the group consisting of H, substituted or unsubstituted C₁ to C₆ alkyl and substituted or unsubstituted C₆ to C₁₄ aryl groups;
X is C-R⁵ or O, wherein R⁵ is selected from the group consisting of H, substituted or unsubstituted C₁ to C₆ alkyl groups and substituted or unsubstituted C₆ to C₁₄ aryl groups, wherein the dashed line represents an optional double bond, if X is C-R⁵;
or a salt of said compound according to formula (II).

3. The pharmaceutical composition for the use according to claim 1 or 2, wherein the compound is further specified in that
R¹ and R² are independently selected from the group consisting of H, F, Cl, Br, I, methyl, ethyl, propyl groups, butyl groups, pentyl groups and phenyl;
R³ and R⁴ are independently selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups;
and R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups, if X is C-R⁵.

4. The pharmaceutical composition for the use according to any one of the preceding claims, wherein the compound is further specified in that R¹ and R² are independently selected from the group consisting of F, Cl, Br, I, methyl, ethyl, propyl groups, butyl groups, pentyl groups and phenyl, preferably from the group consisting of F, Cl, Br, methyl, ethyl, phenyl, more preferably from the group consisting of F, Cl and methyl, even more preferably from the group consisting of Cl and methyl, and most preferably are both Cl.

5. The pharmaceutical composition for the use according to any one of the preceding claims, wherein R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R⁴ preferably is selected from the group consisting of H, methyl, -CN or -C(O)NR⁹R¹⁰ with R⁹ and R¹⁰ being both H, and most preferably R⁴ is -CN;
or in that R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups, wherein R³ preferably is selected from the group consisting of H, methyl, -CN or -C(O)NR⁹R¹⁰ with R⁹ and R¹⁰ being both H, and most preferably R³ is -CN.

6. The pharmaceutical composition for the use according to any one of the preceding claims, wherein the compound is further specified in that
X is C-R⁵, wherein R⁵ is selected from the group consisting of H, methyl, ethyl, propyl groups, butyl groups, pentyl groups, trifluoromethyl and phenyl, preferably from the group consisting of H, methyl, ethyl, n-propyl, trifluoromethyl and phenyl, more preferably is H or methyl, and most preferably is methyl;
and in that the dashed line preferably represents a double bond.

7. The pharmaceutical composition for the use according to any one of the preceding claims, wherein the compound is further specified in that
in that R¹ and R² are independently selected from the group consisting of F, Cl and methyl and preferably are both Cl; and
in that R³ is -CN and R⁴ is selected from the group consisting of H, methyl, -CN, 1-tetrazole, -C(O)NR⁹R¹⁰, wherein R⁹ and R¹⁰ are both H, preferably R⁴ is selected from the group consisting H, -CN and -C(O)NR⁹R¹⁰, and most preferably is -CN,
or R⁴ is -CN and R³ is selected from the group consisting of H, methyl, -CN, 1-tetrazole, -C(O)NR⁹R¹⁰, wherein R⁹ and R¹⁰ are both H, preferably R³ is selected from the group consisting H, -CN and -C(O)NR⁹R¹⁰, and most preferably is -CN; and
in that X is C-R⁵, wherein R⁵ is selected from the group consisting of H, methyl, ethyl, n-propyl, trifluoromethyl and phenyl, more preferably from the group consisting of H and methyl, and most preferably is methyl; and
in that the dashed line preferably represents a double bond.

8. The pharmaceutical composition for the use according to any one of the preceding claims, wherein the compound is selected from the group consisting of the following compounds 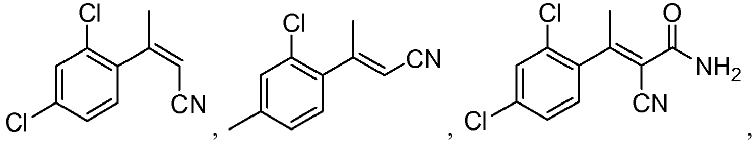 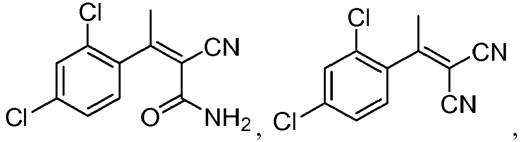 and salts thereof.

9. The pharmaceutical composition for the use according to any one of the preceding claims, wherein the medicament is a GABA_{A} receptor response modulator, especially a positive allosteric GABA_{A} receptor modulator.

10. The pharmaceutical composition for the use according to any one of the preceding claims, wherein modulation of the GABA_{A} receptor response involves the interaction of the compound of the composition with the β subunit(s), especially the β2 and/or β3 isoform(s) of the β subunit(s) of the GABA_{A} receptor.

11. The pharmaceutical composition for the use according to any one of the preceding claims, wherein the modulation of the GABA_{A} receptor response involves the interaction of the compound of the composition with the amino acid β_{2N265} of the β2 subunit and/or the amino acid β_{3N265} of the β3 subunit.

12. A compound of the general formula (I) 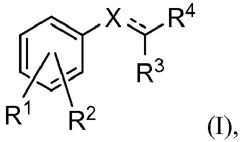 wherein
R¹ is Cl and R² is selected from the group consisting of halogen atoms, substituted or unsubstituted C₁ to C₆ alkyl groups and substituted or unsubstituted C₆ to C₁₄ aryl groups; and
wherein R³ is -CN and R⁴ is selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups, wherein R⁶ to R¹³ are independently selected from the group consisting of H, substituted or unsubstituted C₁ to C₆ alkyl and substituted or unsubstituted C₆ to C₁₄ aryl groups,
or R⁴ is -CN and R³ is selected from the group consisting of H, -CN, -C(O)R⁶, -C(O)OR⁷, -OC(O)R⁸, -C(O)NR⁹R¹⁰, -NR¹¹R¹², -OR¹³, halogen atoms, substituted or unsubstituted C₁ to C₆ alkyl groups, substituted or unsubstituted C₃ to C₆ cycloalkyl groups, substituted or unsubstituted C₆ to C₁₄ aryl groups and substituted or unsubstituted heterocyclic groups, wherein R⁶ to R¹³ are independently selected from the group consisting of H, substituted or unsubstituted C₁ to C₆ alkyl and substituted or unsubstituted C₆ to C₁₄ aryl groups; and
wherein X is C-R⁵, wherein R⁵ is selected from the group consisting of H, substituted or unsubstituted C₁ to C₆ alkyl groups and substituted or unsubstituted C₆ to C₁₄ aryl groups; and
wherein the dashed line represents a double bond;
or a salt of said compound according to formula (I).

13. The compound according to claim 12, wherein the compound is selected from the general formula (II) 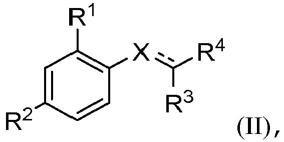 wherein
R¹ is Cl and R² is selected from the group consisting of halogen atoms, substituted or unsubstituted C₁ to C₆ alkyl groups and substituted or unsubstituted C₆ to C₁₄ aryl groups; and
wherein R³ is -CN and R⁴ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups,
or R⁴ is -CN and R³ is selected from the group consisting of H, -CN, -C(O)OR⁷, 1-tetrazole, -C(O)NR⁹R¹⁰, methyl, ethyl, propyl groups, butyl groups, pentyl groups and phenyl, wherein R⁷, R⁹ and R¹⁰ are independently selected from the group consisting of H, substituted or unsubstituted C₁ to C₆ alkyl groups and substituted or unsubstituted C₆ to C₁₄ aryl groups; and
wherein X is C-R⁵, wherein R⁵ is selected from the group consisting of H, substituted or unsubstituted C₁ to C₆ alkyl groups and substituted or unsubstituted C₆ to C₁₄ aryl groups; and
wherein the dashed line represents a double bond;
or a salt of said compound according to formula (II).

14. The compound according to claim 12 or 13, wherein
R² is selected from the group consisting of halogen atoms or unsubstituted C₁ to C₆ alkyl groups, preferably is Cl or methyl, and most preferably is Cl; and
wherein R³ is -CN and R⁴ is selected from the group consisting of H, methyl, -CN, 1-tetrazole, -C(O)NR⁹R¹⁰, wherein R⁹ and R¹⁰ are both H, preferably R⁴ is selected from the group consisting of H, -CN and -C(O)NR⁹R¹⁰, and most preferably R⁴ is -CN,
or R⁴ is -CN and R³ is selected from the group consisting of H, methyl, -CN, 1-tetrazole, -C(O)NR⁹R¹⁰, wherein R⁹ and R¹⁰ are both H, preferably R³ is selected from the group consisting of H, -CN and -C(O)NR⁹R¹⁰, and most preferably R³ is -CN; and
wherein R⁵ is selected from the group consisting of H, unsubstituted C₁ to C₆ alkyl groups, C₁ to C₆ haloalkyl groups, especially C₁ to C₆ fluoroalkyl groups, and unsubstituted C₆ to C₁₄ aryl groups.

15. A pharmaceutical composition according to any one of claims 1 to 11 for treating pain, mood and panic disorders, insomnia, anxiety, and/or neurologic disease, especially migraine and/or epilepsy.
